# EUROPEAN PATENT APPLICATION

(11) **EP 4 703 804 A1**
(43) Date of publication of application: **04.03.2026**
(21) Application number: 25193709.0
(22) Date of filing: 04.08.2025
(51) Int. Cl.: G03G 15/00, G06F 3/12

(54) **INFORMATION PROCESSING SYSTEM, CONTROL METHOD THEREFOR, AND CONTROL PROGRAM THEREFOR**

(30) Priority: 27.08.2024 JP 2024145483
(71) Applicant: Canon Kabushiki Kaisha, Tokyo, 146-8501 (JP)
(72) Inventor: SHIBATA, Daisuke, Tokyo (JP)
(74) Representative: WESER & Kollegen Patentanwälte PartmbB

(57) **Abstract**

An information processing system (1) has a memory device (492) that stores instructions, and a processor (491) that executes the instructions to obtain first information (600a to 600f) related to a process until a primary product, which is a printed matter obtained by an image forming apparatus (101), is output, obtain second information (610a to 610e, 620a to 620e) related to a process until a second product, which is obtained by a postprocessing apparatus (102, 103) processing the primary product, is output, calculate (S906) a CO2 emission emitted from the image forming apparatus based on the first information, calculate (S908, S910) a CO2 emission emitted from the postprocessing apparatus based on the second information, and calculate (S912) a total CO2 emission based on the CO2 emission emitted from the image forming apparatus and the CO2 emission emitted from the postprocessing apparatus.

## Description

### TECHNICAL FIELD

The aspect of the embodiments relates to an information processing system that processes information between an image forming apparatus and a postprocessing apparatus, a control method therefor, and a control program therefor.

### BACKGROUND

In recent years, there is a tendency to calculate an emission of greenhouse gas such as carbon dioxide (CO2) in order to visualize a degree of environmental load. There is a technique of calculating a greenhouse gas emission of an image forming apparatus such as a printer that generates a printed matter by consuming electric power and using coloring material such as ink or toner in printing. For example, Japanese Patent Laid-Open No. 2006-21414 (JP2006-21414A) discloses a technique for calculating an environmental load value representing an environmental load of an image forming apparatus. Specifically, a coloring material consumption, a sheet consumption, and an electric power consumption are obtained on the basis of document data for image formation and job information defining a state of document formation, and the environmental load value representing the environmental load caused by these consumptions is calculated.

As described above, the technique described in the above patent document calculates the environmental load value in obtaining a printed matter by forming an image. Further, the printed matter may be subjected to a postprocess, such as surface treatment like lamination or cutting. In this case, the greenhouse gas may be emitted in the postprocess. However, the technique described in the above patent document cannot calculate the total emission of the greenhouse gas emitted by the image formation and the postprocess.

### SUMMARY

The present disclosure provides an information processing system, a control method therefor, and a control program therefor, which are capable of obtaining the total emission of greenhouse gas emitted until a secondary product is obtained by processing a primary product that is a printed matter.

The present disclosure in its first aspect provides an information processing system as specified in claim 1. Optional features are specified in claims 2 to 13.

The present disclosure in its second aspect provides a control method as specified in claim 14.

The present disclosure in its third aspect provides a control program as specified in claim 15.

According to the present disclosure, it is possible to obtain the total emission of greenhouse gas emitted until a secondary product is obtained by processing a primary product that is a printed matter.

Features of the present disclosure will become apparent from the following description of embodiments with reference to the attached drawings. The following description of embodiments is described by way of example.

### BRIEF DESCRIPTION OF THE DRAWINGS

FIG. 1 is a block diagram illustrating an entire configuration of an information processing system related to a first embodiment.
FIG. 2 is a schematic side view mainly illustrating an internal configuration of an image forming apparatus.
FIG. 3A and FIG. 3B are schematic side views mainly illustrating internal configurations of postprocessing apparatuses.
FIG. 4A is a block diagram illustrating an example of a hardware configuration of the image forming apparatus.
FIG. 4B is a block diagram illustrating an example of a hardware configuration of a surface treatment apparatus.
FIG. 4C is a block diagram illustrating an example of a hardware configuration of a cutting/bookbinding apparatus.
FIG. 4D is a block diagram illustrating an example of a hardware configuration of an environmental load calculation server.
FIG. 5A is a view illustrating an example of a job history screen listing jobs that can be a target of environmental load calculation in the image forming apparatus and the cutting/bookbinding apparatus.
FIG. 5B is a view illustrating an example of a detailed calculation result screen showing a CO2 emission in executing a job selected by a checkbox in FIG. 5A.
FIG. 5C is a view illustrating an example of a calculation result list screen that lists calculation results of CO2 emissions.
FIG. 6A is a view illustrating print job history information required to calculate a CO2 emission in the print process transmitted from the image forming apparatus to the environmental load calculation server.
FIG. 6B is a view illustrating surface-treatment job history information required for calculating a CO2 emission in the surface treatment process transmitted from the surface treatment apparatus to the environmental load calculation server.
FIG. 6C is a view illustrating cutting/bookbinding job history information required for calculating a CO2 emission in the cutting/bookbinding process transmitted from the cutting/bookbinding apparatus to the environmental load calculation server.
FIG. 7 is a block diagram illustrating an example of a software configuration of the environmental load calculation server.
FIG. 8A is a view to describe a relationship between cutting of a sheet on a fore edge side and a waste part (surplus part) generated by the cutting.
FIG. 8B is a view to describe a relationship between cutting of a sheet on the fore edge side, a top side, and a bottom side and waste parts (surplus parts) generated by the cutting.
FIG. 9 is a flowchart illustrating a process executed by the image forming apparatus.
FIG. 10 is a flowchart illustrating a process executed by the postprocessing apparatus.
FIG. 11 is a flowchart illustrating a process executed by the environmental load calculation server.
FIG. 12A is a view illustrating an example of a history screen listing jobs that can be a target of the environmental load calculation in the image forming apparatus and the cutting/bookbinding apparatus related to a second embodiment.
FIG. 12B is a view illustrating an example of a display screen on which a job history of the surface treatment process is input.
FIG. 12C is a view illustrating an example of a display screen on which a job history of the cutting/bookbinding process is input.
FIG. 13 is a flowchart illustrating a process executed by the environmental load calculation server.
FIG. 14 is a flowchart illustrating a process executed by an environmental load calculation server related to a third embodiment.
FIG. 15 is a flowchart illustrating a process (subroutine) executed in a step 1401 of the flowchart illustrated in FIG. 14.

### DESCRIPTION OF THE EMBODIMENTS

Hereinafter, embodiments of the present invention will be described in detail with reference to the drawings. However, the configurations described in the following embodiments are merely examples, and the scope of the present disclosure is not limited by the configurations described in the embodiments. For example, each unit constituting the present disclosure can be replaced with any configuration capable of exhibiting the same function. In addition, an arbitrary constituent may be added. Any two or more configurations (features) of the embodiments can be combined.

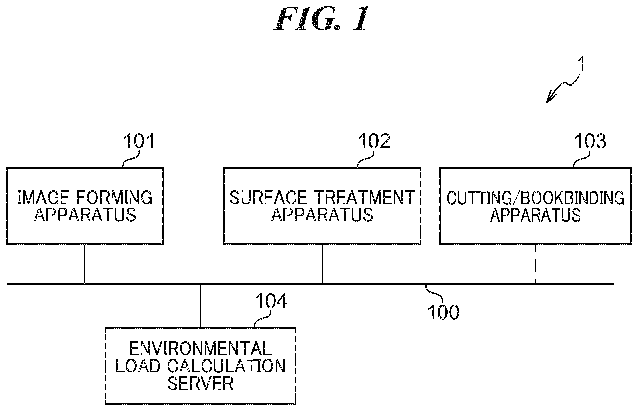

Hereinafter, a first embodiment will be described with reference to FIG. 1 to FIG. 11. FIG. 1 is a block diagram illustrating an entire configuration of an information processing system related to the first embodiment. As illustrated in FIG. 1, the information processing system 1 includes an image forming apparatus 101, a surface treatment apparatus 102, a cutting/bookbinding apparatus 103, and an environmental load calculation server 104, which are communicably connected to each other via a network 100.

The information processing system 1 calculates (computes) environmental load amounts of the image forming apparatus 101, the surface treatment apparatus 102, and the cutting/bookbinding apparatus 103. Here, the "environmental load" is a negative influence on the environment, and is, for example, greenhouse gases in the present embodiment. The greenhouse gases include carbon dioxide (CO2), methane (CH4), nitrous oxide (N2O). In the present embodiment, CO2 will be described as a representative example. The image forming apparatus 101 receives a print job from an external apparatus (not shown) via the network 100. The image forming apparatus 101 outputs a printed matter as a primary product by printing on a print medium based on the print job.

The surface treatment apparatus 102 and the cutting/bookbinding apparatus 103 are postprocessing apparatuses each capable of outputting a secondary product by performing process on a primary product. Although the surface treatment apparatus 102 and the cutting/bookbinding apparatus 103 are included as the postprocessing apparatuses in the present embodiment, it is enough that at least one of the surface treatment apparatus 102 and the cutting/bookbinding apparatus 103 is included.

The surface treatment apparatus 102 performs treatment on a surface of a primary product. Although the surface treatment apparatus 102 performs the surface treatment of applying varnish in the present embodiment, this is not limited. For example, the surface treatment may be attaching a film or pressing a foil.

The cutting/bookbinding apparatus 103 cuts the primary product of which the surface has been treated by the surface treatment apparatus 102. A secondary product is obtained by applying the surface treatment and the cutting to the primary product. In the present embodiment, the cutting/bookbinding apparatus 103 can bind the secondary product in a booklet state.

The environmental load calculation server 104 is an information processing apparatus that processes information exchanged with the image forming apparatus 101, information exchanged with the surface treatment apparatus 102, and information exchanged with the cutting/bookbinding apparatus 103. In the present embodiment, the environmental load calculation server 104 can calculate the environmental load amount related to the product generated by the image forming apparatus 101, the surface treatment apparatus 102, and the cutting/bookbinding apparatus 103. The environmental load calculation server 104 is not particularly limited, and for example, a desktop or notebook personal computer or the like is used.

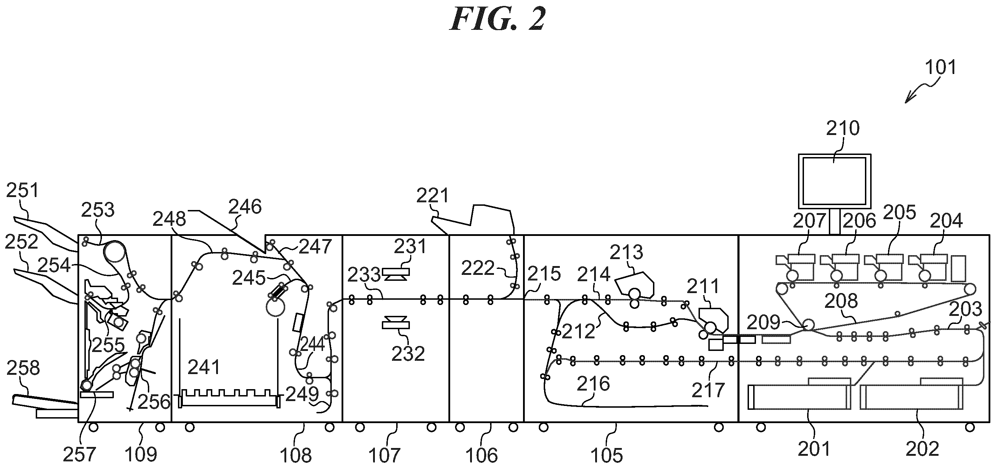

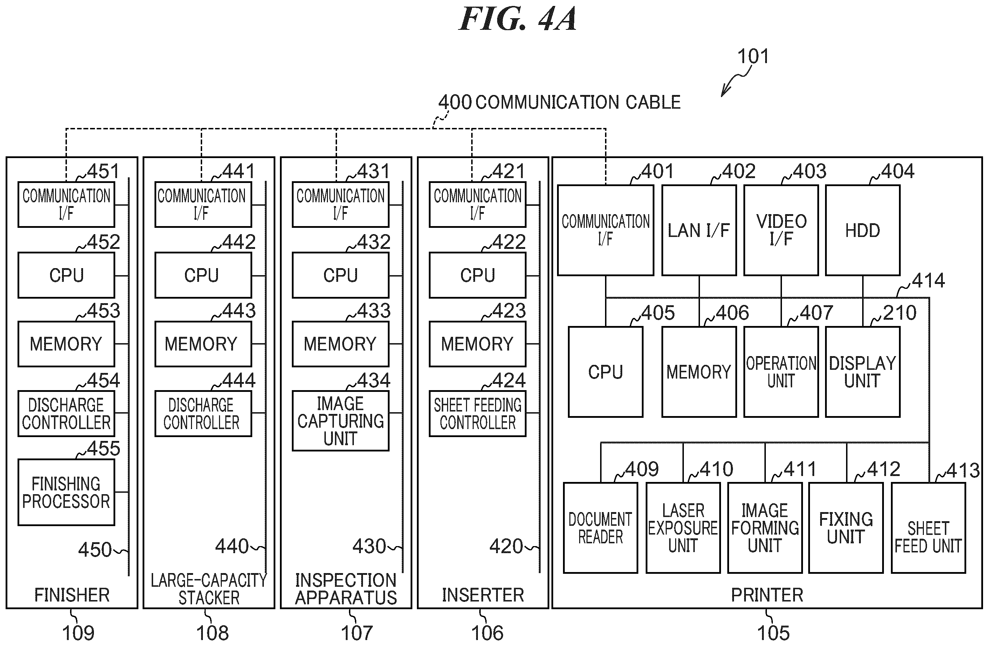

FIG. 2 is a schematic side view mainly illustrating an internal configuration of the image forming apparatus. As illustrated in FIG. 2, the image forming apparatus 101 includes a printer 105, an inserter 106, an inspection apparatus 107, a large-capacity stacker 108, and a finisher 109, which are arranged in the order from an upstream side toward a downstream side in a conveyance direction of a print medium. The printer 105 prints and forms an image on a sheet. The printer 105 includes sheet feed decks 201 and 202, development stations 204 to 207, an intermediate transfer belt 208, a secondary transfer position 209, and a display unit 210. Various types of sheets (print sheets), which are print media, can be stored in the paper feed decks 201 and 202. In printing, information such as sizes of the sheets stored in the sheet feed decks 201 and 202 can be set on an operation unit 407 (see FIG. 4A) of the image forming apparatus 101. Based on this setting, the uppermost sheet is conveyed one by one from the sheet feed deck 201 or 202 to a sheet conveyance path 203. The operation unit 407 can also display a print status of the image forming apparatus 101 on the display unit 210.

The development station 204 forms a toner image using yellow (Y) toner. The development station 205 forms a toner image using magenta (M) toner. The development station 206 forms a toner image using cyan (C) toner. The development station 207 forms a toner image using black (K) toner. The toner images of the respective colors are primarily transferred to the intermediate transfer belt 208 rotating clockwise in FIG. 2 in an overlapped fashion. At the secondary transfer position 209, the toner images on the intermediate transfer belt 208 are transferred onto a sheet conveyed from the sheet conveyance path 203. Thus, a color image can be formed on the sheet.

The printer 105 includes a first fixing unit 211 and a second fixing unit 213. The first fixing unit 211 includes a pressure roller and a heating roller, and can fix a toner image on a sheet by melting and pressing toner when the sheet passes between the rollers. The sheet that has passed through the first fixing unit 211 passes through a sheet conveyance path 212 and is conveyed to a sheet conveyance path 215.

Some types of sheets further require melting and pressing to fix the toner image. In this case, the sheet is conveyed to the second fixing unit 213 after passing through the first fixing unit 211. The second fixing unit 213 applies additional melting and pressing to the sheet. Thereafter, the sheet is conveyed to the sheet conveyance path 215 through a sheet conveyance path 214. In a case of two-sided printing, the sheet is conveyed to a sheet reversing path 216 to be reversed, and then conveyed to a duplex conveying path 217, and an image is transferred to the back side at the secondary transfer position 209.

The inserter 106 inserts a sheet (hereinafter, "an insertion sheet") at an arbitrary position in a sheet group including a plurality of sheets printed by the printer 105. The inserter 106 includes an inserter tray 221. An insertion sheet conveyed from the inserter tray 221 through a sheet conveyance path 222 is inserted into a sheet group fed from the printer 105. The sheet group into which the insertion sheet is inserted is conveyed to the inspection apparatus 107.

The inspection apparatus 107 includes cameras 231 and 232 that are arranged to face each other. The camera 231 captures an image of a front side of a sheet. The camera 232 captures an image of a back side of a sheet. The inspection apparatus 107 captures an image of a front side of a sheet with the camera 231 and captures an image of a back side of the sheet with the camera 232 at a timing when the sheet conveyed along the sheet conveyance path 233 reaches a predetermined position. This enables to read images printed on a sheet of the sheet group conveyed from the inserter 106.

The inspection apparatus 107 compares the read image with a reference image stored in advance, and inspects whether printing by the printer 105 has been performed without any problem. As a result of the inspection, a sheet group determined to have no problem in printing is conveyed to the large-capacity stacker 108 as a normal printed matter. On the other hand, a sheet group determined to have a problem in printing, which is an unnecessary primary product, is discharged separately from the normal printed matter.

The large-capacity stacker 108 includes a stack tray 241 as a tray on which normal printed matters are stacked. A large capacity of sheets that have passed through a sheet conveyance path 244 and a sheet conveyance path 245 in this order from the inspection apparatus 107 are stacked on the stack tray 241. The large-capacity stacker 108 also includes an escape tray 246 as a discharge tray. A sheet group determined to have a problem in printing by the inspection apparatus 107 is stacked on the escape tray 246. In this case, the sheets of the sheet group determined to have a problem pass through the sheet conveyance path 244 and a sheet conveyance path 247 in this order, and are conveyed to the escape tray 246. When a sheet is conveyed from the large-capacity stacker 108 to a postprocessing apparatus, the sheet passes through a sheet conveyance path 248.

A sheet conveyed from the large-capacity stacker 108 to the postprocessing apparatus is a primary product. The large-capacity stacker 108 includes a reversing unit 249 that reverses a sheet to match an orientation of the sheet in inputting and the orientation of the sheet in outputting. The reversing unit 249 is used when stacking the sheet on the stack tray 241. The reversing unit 249 is not used when the sheet is conveyed to the escape tray 246 or the postprocessing apparatus.

The finisher 109 performs a finishing process on a sheet in accordance with the setting in the operation unit 407. The finishing process is not particularly limited, and s thereof include stapling to bind sheets at one or two positions and punching to form two or three holes, for example. The finisher 109 includes sheet discharge trays 251 and 252, and a processing unit 255.

A sheet is conveyed to the sheet discharge tray 251 passing through a sheet conveyance path 253. In this case, the finishing process for the sheet is omitted.

A sheet is conveyed to the sheet discharge tray 252 passing through a sheet conveyance pass 254. In this case, the processing unit 255 performs the finishing processing on the sheet. The paper discharge trays 251 and 252 are each capable of elevating.

The finisher 109 includes a saddle stitching unit 256 and a saddle stitching bookbinding tray 258. In this case, saddle stitching can be performed as the finishing process. After the stapling process is performed on the center of the sheets by the processing unit 255, the sheets are folded in two by the saddle stitching processing unit 256 to be in a saddle stitching bookbinding state, and pass through the sheet conveyance path 257. After that, the sheets in the saddle stitch bookbinding state is conveyed to the saddle stitching bookbinding tray 258. The saddle stitching bookbinding tray 258 includes a belt conveyor. As a result, the sheets in the saddle stitch bookbinding state placed on the belt conveyor is conveyed leftward in FIG. 2.

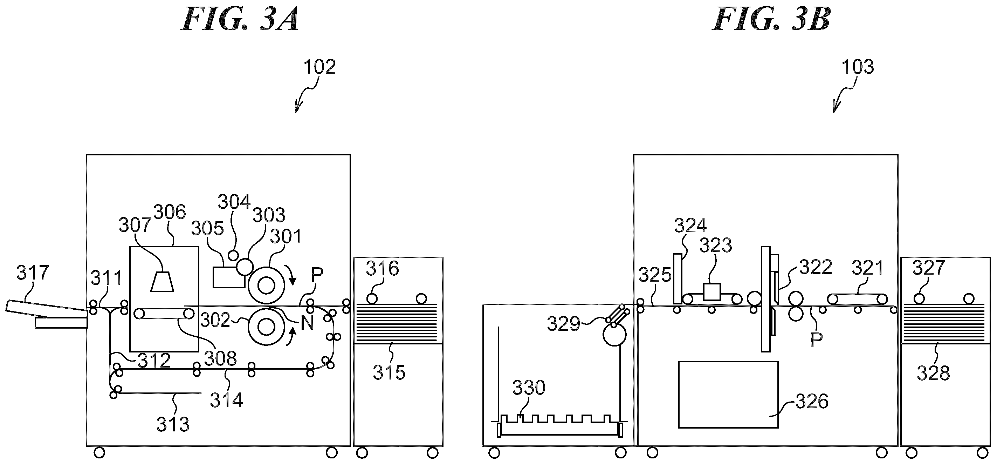

FIG. 3A is a schematic side view mainly illustrating an internal configuration of the surface treatment apparatus 102. FIG. 3B is a schematic side view mainly illustrating an internal configuration of the cutting/bookbinding apparatus 103. The surface treatment apparatus 102 shown in FIG. 3A is a roll-type varnishing coater that coats the primary product with varnishing liquid. The surface treatment apparatus 102 includes a coating roller 301, a backup roller 302, a feed roller 303, a regulation roller 304, a storage portion 305, and a curing device 306. The coating roller 301 and the backup roller 302 form a coating nip portion N for coating a sheet P as a primary product with the varnishing liquid.

The sheet P is fed from a sheet feed tray 315 by a sheet feed roller 316, and is discharged to a discharge tray 317 after the varnish liquid is coated. The varnish liquid is stored in the storage portion 305. A part of the surface of the feed roller 303 is immersed in the varnish liquid stored in the storage portion 305. The varnish liquid is drawn up from the storage portion 305 by the rotation of the feed roller 303, passes through a facing portion between the feed roller 303 and the regulation roller 304, and then moves to the surface of the coating roller 301 resulting from contact between the feed roller 303 and the coating roller 301. The amount of the varnish liquid applied to the coating roller 301 (film thickness) can be adjusted by adjusting at least one of the rotation speed of the feed roller 303, the distance between the feed roller 303 and the regulation roller 304, and the contact pressure between the feed roller 303 and the coating roller 301.

The curing device 306 includes a curing unit 307 and a conveyance unit 308. The curing unit 307 can cure or dry the varnish liquid by UV irradiation, heating, or the like depending on the type of the varnish liquid. In a case where the varnish liquid is coated on the front surface of the sheet P, the sheet P passes through the curing device 306, further passes through an external discharge path 311, and is discharged to the discharge tray 317.

In a case where the varnish liquid is coated on the back surface of the sheet P, the sheet P passes through a conveyance path 312, a reversing path 313, and a two-sided conveyance path 314 in this order, and is reversed. Then, the varnish liquid is coated on the back surface of the sheet P through the same process as the case where the varnish liquid is coated on the front surface of the sheet P. The sheet P of which the both surfaces are coated with the varnish liquid passes through the curing device 306 again, then passes through the external discharge path 311 and is discharged to the discharge tray 317.

The cutting/bookbinding apparatus 103 shown in FIG. 3B is a three way cutter capable of cutting a sheet in three directions. The cutting/bookbinding apparatus 103 includes a conveyance unit 321, a cutter unit 322, a press unit 323, an abutment portion 324, a conveyance unit 325, a waste box 326, a sheet feed roller 327, a sheet feed tray 328, a sheet discharge roller 329, and a sheet discharge tray 330.

In the cutting/bookbinding apparatus 103, the sheet P on the sheet feed tray 328 is fed by the sheet feed roller 327. The sheet P is conveyed to the cutting position by the conveyance unit 321, and is positioned by the abutment portion 324. The cutter unit 322 descends in a state where the sheet P is fixed by the press unit 323. Thus, the cutter unit 322 cuts the sheet P along a line orthogonal to the conveyance direction. A cut piece of the sheet P generated by the cutting falls by its own weight and is stored in the waste box 326.

The cutter unit 322 includes an adjustment mechanism that adjusts a cutting position of the sheet P in the conveyance direction. In addition to the cutter unit 322, the cutting/bookbinding apparatus 103 includes cutter units (not illustrated) on both sides of the sheet P in a width direction orthogonal to the conveyance direction to cut the sheet P along lines parallel to the conveyance direction. Each of the cutter units includes an adjustment mechanism that adjusts a cutting position in the width direction. These three cutter units enable, for example, fore edge cutting and three side cutting. The sheet P is conveyed by the conveyance unit 325 after the cutting, and is discharged to the discharge tray 330 by the discharge roller 329.

Although the cutting/bookbinding apparatus 103 is configured to convey and cut the sheet P one by one in the present embodiment, this is not limited. For example, the cutting/bookbinding apparatus 103 may be configured to convey and cut a plurality of sheets P or configured to convey and cut in units of sheet groups.

FIG. 4A is a block diagram showing an example of a hardware configuration of the image forming apparatus 101. As illustrated in FIG. 4A, the printer 105 includes a communication I/F 401, a LAN I/F 402, a video I/F 403, an HDD 404, a CPU 405, a memory 406, the operation unit 407, and the display unit 210. The printer 105 includes a document reader 409, a laser exposure unit 410, an image forming unit 411, a fixing unit 412, and a sheet feed unit 413. These hardware components included in the printer 105 are communicably connected to each other via a system bus 414.

The communication I/F 401 is communicably connected to a communication I/F 421 of the inserter 106, a communication I/F 431 of the inspection apparatus 107, a communication I/F 441 of the large-capacity stacker 108, and a communication I/F 451 of the finisher 109 via a communication cable 400. The LAN I/F 402 is communicably connected to a print server (not shown) and an information processing apparatus (not shown) via the network 100 and receives print instructions from them.

The LAN I/F 402 is communicably connected to the environmental load calculation server 104 to enable communication of job history information. A job ID to identify the job history information may be assigned by the image forming apparatus 101, may be designated by the print server or the information processing apparatus, or may be set by a user input. In addition, when a print instruction is received from the print server or the information processing apparatus, job IDs of processes for each product may be recorded in association with each other, or a print instruction using the same job ID as the above-mentioned job ID may be issued.

The video I/F 403 is communicably connected to a PC (not shown) or an external controller (not shown) that generates a print image via a video cable (not shown), and performs communication of rasterized image data. The HDD 404 is a storage device that stores programs and data. The CPU 405 comprehensively performs, for example, image process control and print control based on programs stored in the HDD 404.

The memory 406 stores programs and image data necessary for the CPU 405 to perform various processes, and operates as a work area of the CPU 405. The operation unit 407 accepts inputs about various settings and an instruction for an operation from a user. The display unit 210 displays, for example, setting information about the printer 105 and a processing status of a print job.

The document reader 409 performs processing for reading a document when using a copy function or a scan function. In the present embodiment, the document reader 409 reads a document by capturing an image with a CCD camera while irradiating a sheet placed by the user with an exposure lamp. The laser exposure unit 410 primarily charges a photosensitive drum and irradiates the drum with a laser beam to form an electrostatic latent image.

Specifically, first, the laser exposure unit 410 performs primary charging to charge the surface of the photosensitive drum to a uniform negative potential. Next, the laser exposure unit 410 irradiates the photosensitive drum with laser beam from a laser driver while changing a reflection angle with a polygon mirror. As a result, the negative charge of the irradiated portion of the photosensitive drum is neutralized, and the electrostatic latent image is formed. The image forming unit 411 includes a developing unit, a transfer unit, and a toner supply unit, and transfers toner on a photosensitive drum to a sheet.

The developing unit causes negatively charged toner to adhere to the electrostatic latent image on the surface of the photosensitive drum from a developing cylinder, thereby visualizing the electrostatic latent image. The transfer unit applies a positive potential to a primary transfer roller to perform primary transfer of transferring the toner on the surface of the photosensitive drum to the transfer belt. The transfer unit applies a positive potential to the secondary transfer roller to perform secondary transfer to transfer the toner on the transfer belt to a sheet.

The fixing unit 412 includes a heater, a fixing belt, and a pressure belt, and melts and fixes the toner on the sheet to the sheet by heat and pressure. The sheet feed unit 413 includes a roller to feed a sheet, and controls a feed operation and a conveying operation of the sheet in accordance with detection results of various sensors.

As illustrated in FIG. 4A, the inserter 106 includes the communication I/F 421, a CPU 422, a memory 423, and a paper feed controller 424, which are communicably connected to each other via a system bus 420. The CPU 422 executes a control program stored in the memory 423 to perform various controls necessary for sheet feeding. The memory 423 is a storage device that stores the control program. The sheet feed controller 235 controls conveyance of a sheet in accordance with an instruction from the CPU 421.

As illustrated in FIG. 4A, the inspection apparatus 107 includes the communication I/F 431, a CPU 432, a memory 433, and an image capturing unit 434, which are communicably connected to each other via a system bus 430. The CPU 432 executes a control program stored in the memory 433 to perform various controls necessary for inspection. The CPU 432 may execute a control program stored in an external server, for example, to perform various controls necessary for the inspection. The memory 433 is a storage device that stores the control program. The memory 433 may store setting contents and a history of execution results of the inspection. The image capturing unit 434 captures an image of the conveyed sheet in accordance with an instruction from the CPU 432. The CPU 432 analyzes the image captured by the image capturing unit 434 to inspect the primary product.

As illustrated in FIG. 4A, the large-capacity stacker 108 includes the communication I/F 441, a CPU 442, a memory 443, and a discharge controller 444, which are communicably connected to each other via a system bus 440. The CPU 442 executes the control program stored in the memory 443 to perform various controls necessary for discharging a sheet. The memory 443 is a storage device that stores the control program. The discharge controller 444 controls to convey a sheet in accordance with an instruction from the CPU 442.

As illustrated in FIG. 4A, the finisher 109 includes the communication I/F 451, a CPU 452, a memory 453, a discharge controller 454, and a finishing processor 455, which are communicably connected to each other via a system bus 450. The CPU 452 executes a control program stored in the memory 453 to perform various controls necessary for finishing and discharging a sheet. The memory 453 is a storage device that stores the control program. The discharge controller 454 controls to convey and discharge a sheet in accordance with an instruction from the CPU 452. The finishing processor 455 controls a finishing process for the primary product after the inspection in accordance with an instruction from the CPU 452.

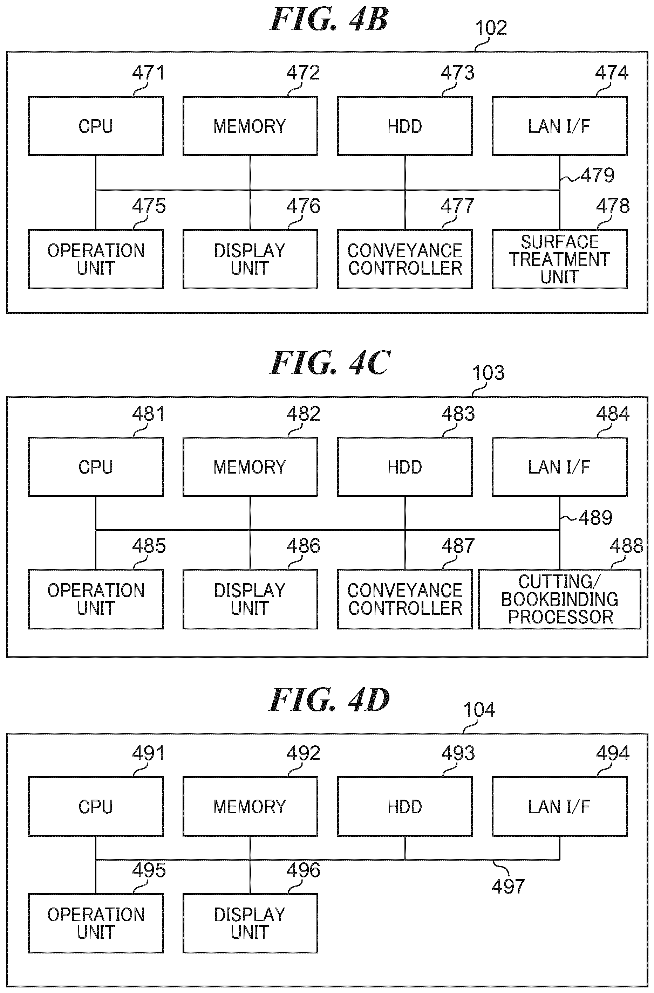

FIG. 4B is a block diagram illustrating an example of a hardware configuration of the surface treatment apparatus 102. FIG. 4C is a block diagram illustrating an example of a hardware configuration of the cutting/bookbinding apparatus 103. FIG. 4D is a block diagram illustrating an example of a hardware configuration of the environmental load calculation server 104.

As illustrated in FIG. 4B, the surface treatment apparatus 102 includes a CPU 471, a memory 472, an HDD 473, a LAN I/F 474, and an operation unit 475. In addition, the surface treatment apparatus 102 includes a display unit 476, a conveyance controller 477, and a surface treatment unit 478. These hardware components included in the surface treatment apparatus 102 are communicably connected to each other via a system bus 479.

The CPU 471 executes a control program stored in the memory 472 to perform various controls necessary for the surface treatment. The control program is recorded in the memory 472 or the HDD 473. The HDD 473 stores set values used in performing the surface treatment process and a job history. The conveyance controller 477 controls conveyance of a sheet in accordance with an instruction from the CPU 471. The surface treatment unit 478 controls the surface treatment process in accordance with an instruction from the CPU 471. The LAN I/F 474 is communicably connected to a print server (not shown) or an information processing apparatus (not shown) via the network 100 and receives a treatment instruction as a job.

The LAN I/F 474 is communicably connected to the environmental load calculation server 104 to enable communication of the job history information. The job ID for identifying the job history information may be assigned by the surface treatment apparatus 102, may be designated by the print server or the information processing apparatus, or may be set by a user input. When a job is received from the print server or the information processing apparatus, a job ID associated with a job for another related apparatus may be assigned. The surface treatment apparatus 102 may be incorporated as a part of the image forming apparatus 101.

As illustrated in FIG. 4C, the cutting/bookbinding apparatus 103 includes a CPU 481, a memory 482, an HDD 483, a LAN I/F 484, and an operation unit 485. In addition, the cutting/bookbinding apparatus 103 includes a display unit 486, a conveyance controller 487, and a cutting/bookbinding processor 488. These hardware components included in the cutting/bookbinding apparatus 103 are communicably connected to each other via a system bus 489.

The CPU 481 executes a control program stored in the memory 482 to perform various controls necessary for cutting and bookbinding. The control program is recorded in the memory 482 or the HDD 483. In addition, the HDD 483 stores set values for performing the cutting/bookbinding process and a job history. The conveyance controller 487 controls conveyance of a sheet in accordance with an instruction from the CPU 481. The cutting/bookbinding processor 488 controls the cutting/bookbinding process in accordance with an instruction from the CPU 481. The LAN I/F 484 is communicably connected to a print server (not shown) or an information processing apparatus (not shown) via the network 100 and receives a treatment instruction as a job.

The LAN I/F 484 is communicably connected to the environmental load calculation server 104 to enable communication of job history information. The job ID to identify the job history information may be assigned by the cutting/bookbinding apparatus 103, may be designated by the print server or the information processing apparatus, or may be set by a user input. When a job is received from the print server or the information processing apparatus, a job ID associated with a job for another related apparatus may be assigned. The cutting/bookbinding apparatus 103 may be incorporated as a part of the image forming apparatus 101.

As illustrated in FIG. 4D, the environmental load calculation server 104 includes a CPU 491, a memory 492, an HDD 493, a LAN I/F 494, an operation unit 495, and a display unit 496, which are communicably connected to each other via a system bus 497. The CPU 491 comprehensively executes processes such as reception of job history information from the image forming apparatus 101, the surface treatment apparatus 102, and the cutting/bookbinding apparatus 103 and calculation of the environmental load amount in accordance with a program stored in the HDD 493.

The memory 492 stores programs and data necessary for the CPU 491 to perform various processes, and operates as a work area of the CPU 491. The HDD 493 stores programs and data necessary for operations of the print process. The operation unit 495 accepts inputs about various settings and an instruction for an operation from a user. On the display unit 496, for example, information about an execution application of the environmental load calculation server 104 is displayed as a still image or a moving image.

The LAN I/F 494 is connected to the image forming apparatus 101, the surface treatment apparatus 102, and the cutting/bookbinding apparatus 103 via the network 100, and performs communication of a job history and the like. In the information processing system 1, each memory such as the memory 492 may be replaced with, for example, a volatile RAM, a nonvolatile ROM, a built-in HDD, an external HDD, a USB memory, or the like. Further, a program for causing each CPU (computer) to execute each unit or each function (a control method) of the information processing system 1 may be stored in one apparatus or may be stored in each apparatus in a distributed manner.

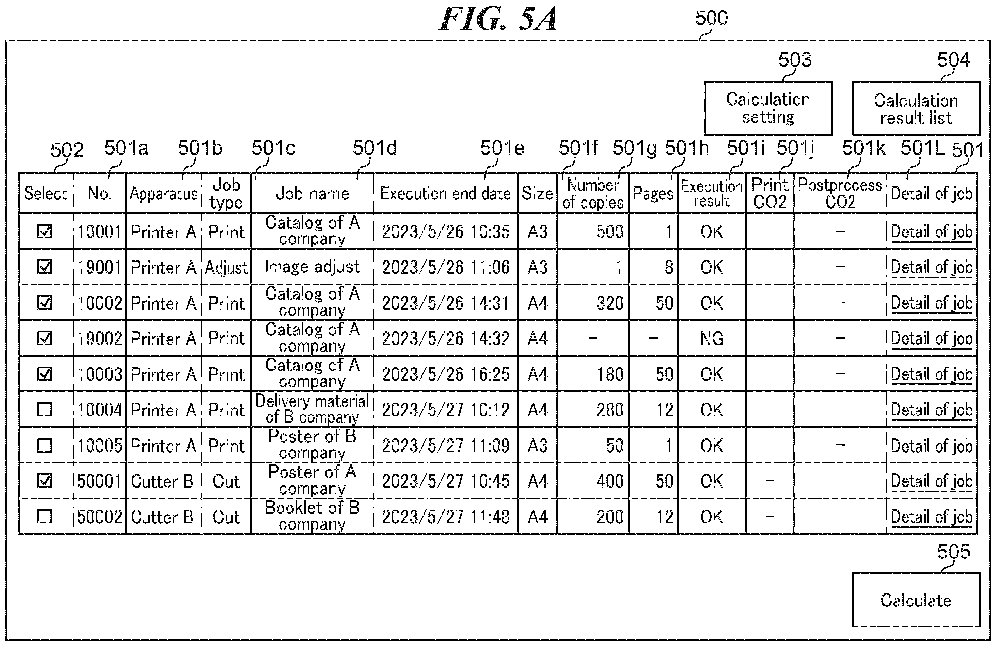

FIG. 5A is a view illustrating an example of a job history screen listing jobs that can be a target of environmental load calculation in the image forming apparatus 101 and the cutting/bookbinding apparatus 103. A job history screen (history screen) 500 shown in FIG. 5A is displayed on the display unit 496 of the environmental load calculation server 104 or the display unit 210 of the printer 105. The setting information on the job history screen 500 is stored in the HDD 493 of the environmental load calculation server 104.

The job history screen 500 includes a job history table 501, a calculation setting button 503, a calculation result list button 504, and a CO2 emission calculation button 505. The job history table 501 includes a column 502 and columns 501a to 501L. A checkbox to select a target job is displayed in the column 502. In the column 501a, a job number to identify a job is described (input). In the column 501b, an apparatus that has executed a job is described. In the column 501c, a type of a job is described. In the column 501d, a name of a job is described. In the column 501e, an execution end date and time of a job is described.

In the column 501f, a size of a sheet used in a job is described. In the column 501g, the number of copies obtained by executing a job is described. In the column 501h, the number of pages obtained by executing a job is described. In the column 501i, an execution result of a job is described. In the column 501j, a CO2 emission generated by printing is described. In the column 501k, a CO2 emission generated by a postprocess is described. In the column 501L, a detail of a job is described.

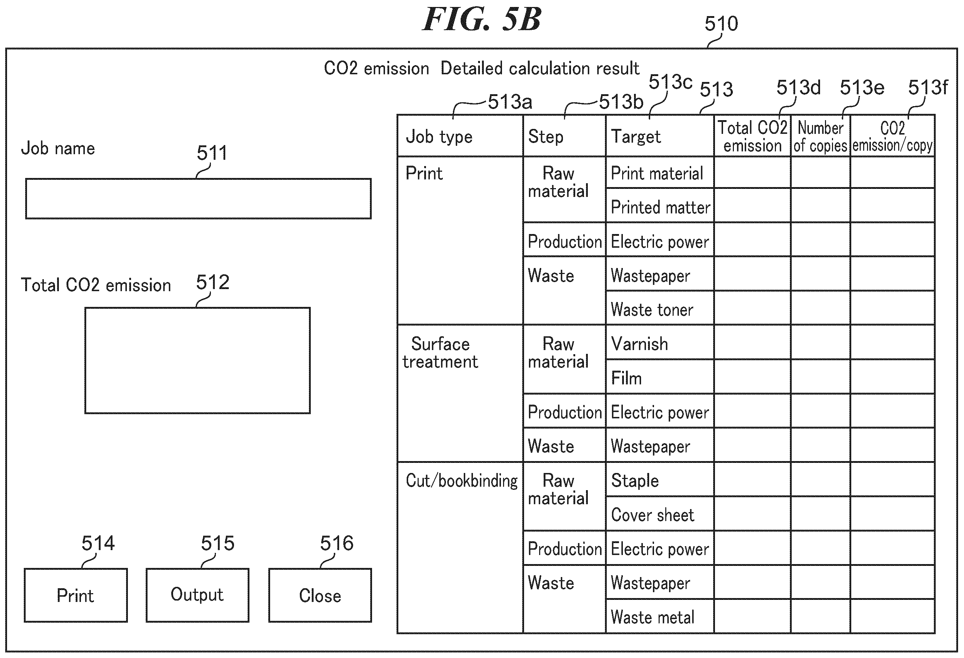

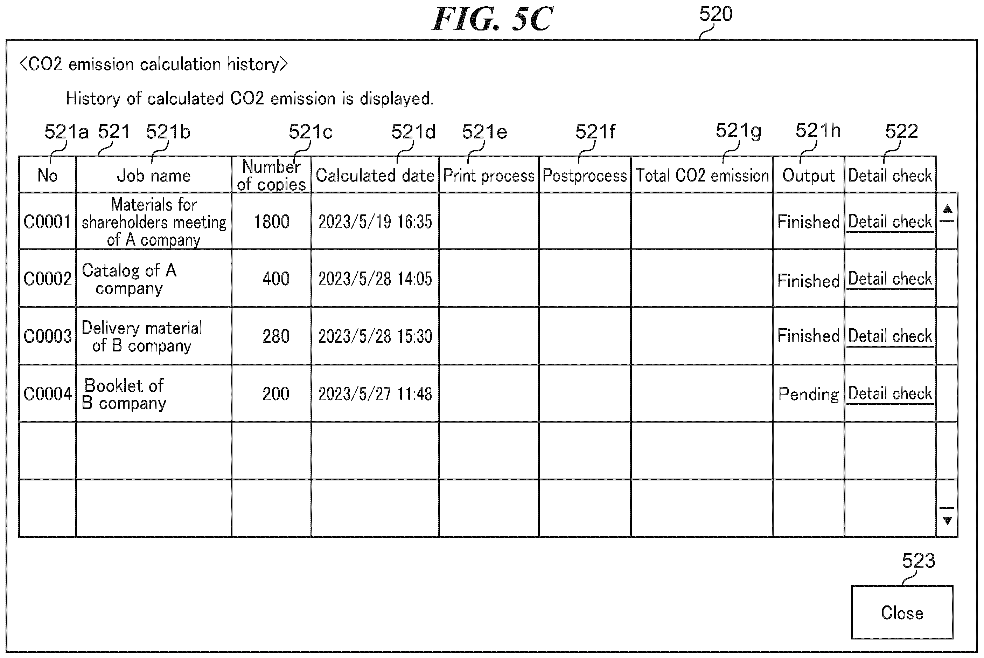

By operating a checkbox in the column 502, a calculation target of the CO2 emission can be selected from the job history table 501 prior to the calculation of the CO2 emission. When the calculation setting button 503 is operated, a screen for setting conditions used for calculation of the CO2 emission can be displayed. When the calculation result list button 504 is operated, a calculation result list screen 520 (see FIG. 5C) indicating a list of calculation results of CO2 emissions can be displayed. An operation of the CO2 emission calculation button 505 instructs to calculate a CO2 emission in a job selected in a checkbox of the column 502. After the execution, a detailed calculation result screen 510 (see FIG. 5B) is displayed.

FIG. 5B is a view illustrating an example of the detailed calculation result screen showing a CO2 emission in executing a job selected by a checkbox in FIG. 5A. The detailed calculation result screen 510 shown in FIG. 5B includes a calculation-target job name field 511, a total CO2 emission display field 512, a detailed calculation result display table 513, a print button 514, an output button 515, and a close button 516.

The calculation-target job name field 511 displays a name of a job (the column 501d) selected by a checkbox of the column 502. The total CO2 emission display field 512 displays the calculation result of the CO2 emission in executing the job selected by the checkbox of the column 502. When a plurality of jobs are selected by the checkboxes of the column 502, the total CO2 emission in executing these jobs is displayed.

The detailed calculation result display table 513 includes columns 513a to 513f. In the column 513a, a type of a job (process) is described. In the column 513b, a CO2 emission calculation step in a job is described. In the column 513c, a CO2 emission calculation target in a job is described. In the column 513d, a total CO2 emission is described. In the column 513e, the number of copies of printed matter is described. In the column 513f, a CO2 emission per a copy is described.

When the print button 514 is operated, for example, the contents described in the calculation-target job name field 511, the total CO2 emission display field 512, and the detailed calculation result display table 513 are printed by the image forming apparatus 101. When the output button 515 is operated, for example, the contents described in the calculation-target job name field 511, the total CO2 emission display field 512, and the detailed calculation result display table 513 are stored as a file in the HDD 493 of the environmental load calculation server 104. When the close button 516 is operated, the detailed calculation result screen 510 is closed. After that, the job history screen 500 is displayed again.

FIG. 5C is a view illustrating an example of the calculation result list screen that lists calculation results of the CO2 emissions. The calculation result list screen 520 includes a calculation history display table 521 and a close button 523. The calculation history display table 521 includes columns 521a to 521h and a column 522. In the column 521a, a management number for identifying the calculation result of CO2 emission is described. In the column 521b, a name of a job is described.

In the column 521c, the number of copies obtained by executing a job is described. In the column 521d, a calculation date of a CO2 emission is described. In the column 521e, a CO2 emission of a print job (print process) is described. In the column 521f, a CO2 emission of a postprocess job (postprocess). In the column 521g, the total CO2 emission that is the sum of the CO2 emission in the column 521e and the CO2 emission in the column 521f is described. In the column 521h, whether the output is finished or pending is described.

When a calculation result check button included in the column 522 is operated, the detailed calculation result screen 510 (see FIG. 5B) is displayed. When the close button 523 is operated, th calculation result list screen 520 is closed. After that, the job history screen 500 is displayed again.

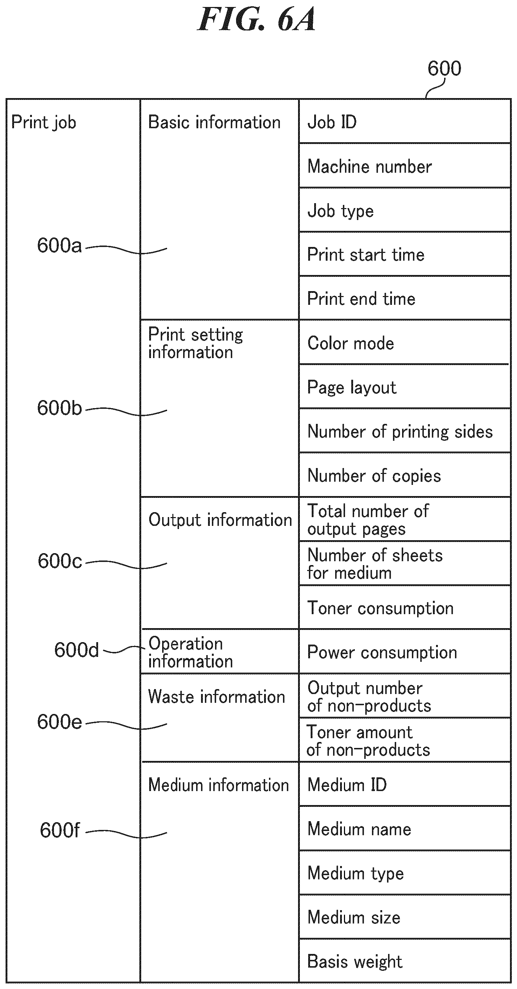

FIG. 6A is a view illustrating print job history information 600 required to calculate a CO2 emission in the print process transmitted from the image forming apparatus to the environmental load calculation server. The print job history information (a first execution history) 600 illustrated in FIG. 6A is stored in the HDD 493 of the environmental load calculation server 104. The print job history information 600 is used as information included in the job history screen 500 and the detailed calculation result screen 510. Although the print job history information 600 includes basic information 600a, print setting information 600b, output information 600c, operation information 600d, waste information 600e, and medium information 600f in the present embodiment, this is not limited.

The basic information 600a includes, for example, a job ID to identify a job. The print setting information 600b includes, for example, a color mode to designate color print or monochrome print, a page layout, the number of sides to be printed on a sheet, and the number of copies obtained by printing. The output information 600c relates to an amount of material used in a process until a primary product is output by the image forming apparatus 101. Specifically, the output information 600c includes the total number of pages obtained by printing, the number of sheets used for each medium, and a toner consumption consumed by printing. The number of sheets for each medium is associated with a medium ID recorded as the medium information. The toner consumption is a value obtained by multiplying a count value of dots formed of the toner by the toner amount per dot.

The operation information 600d is an amount of power used in a process until a primary product is output by the image forming apparatus 101, that is, an electric power consumption (hereinafter referred to as a power consumption) consumed from a print start time to a print end time. The power consumption may be obtained from a power consumption calculation table stored in advance. The power consumption is the total of power consumptions consumed by the apparatuses from the printer 105 to the finisher 109 constituting the image forming apparatus 101.

The waste information 600e relates to an amount of waste material generated in a process until a primary product is output by the image forming apparatus 101. The waste is not particularly limited and includes a sheet determined by the inspection apparatus 107 to have a problem in printing, a sheet that is retained due to a sheet jam in the image forming apparatus 101 and is removed from the image forming apparatus 101, and a sheet used for adjusting the image forming apparatus 101. The number of such sheets is counted as the output number of non-products. Another example of the waste is toner used for printing on a sheet that has become waste. Such an amount of toner is processed as a toner amount of non-products.

The medium information 600f includes, for example, a medium ID of a medium (sheet) used for printing, a medium name, a medium type, a medium size, and a basis weight of a medium. The medium ID is an ID for identifying a medium (sheet). The medium type is, for example, a type of a sheet, such as wood-free paper, coated paper, or recycled paper. The medium size is a classification of a prescribed size, a length in a width direction, and a length in a conveyance direction. When a length in the width direction and a length in the conveyance direction, which are not included in any classifications of prescribed sizes, are designated, the medium size is recorded as a user definition. The basis weight is the weight of a sheet per square meter. The basic information 600a to the medium information 600f as described above are the information related to the process until the primary product is output by the image forming apparatus 101. Hereinafter, the information may be referred to as "first information".

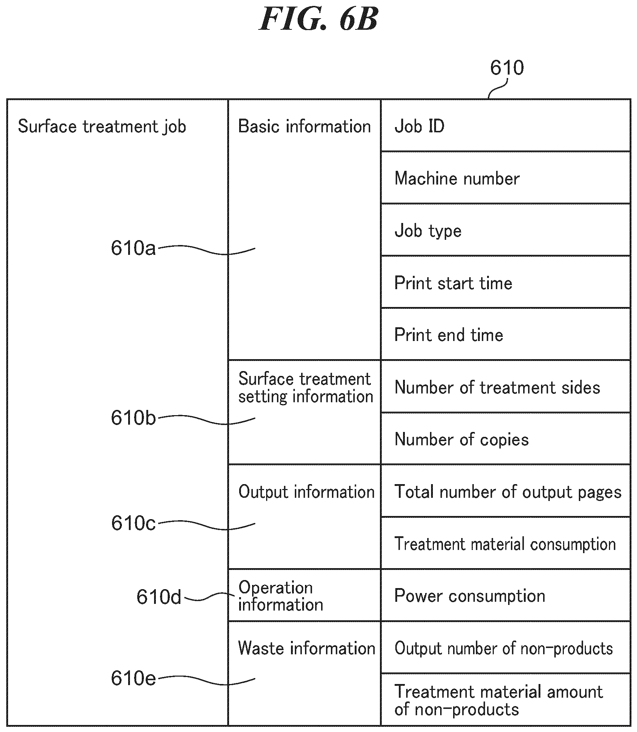

FIG. 6B is a view illustrating surface-treatment job history information 610 required for calculating a CO2 emission in the surface treatment process among the postprocesses. The surface-treatment job history information (second execution history) 610 illustrated in FIG. 6B is stored in the HDD of the environmental load calculation server 104. The surface-treatment job history information 610 is used as information included in the job history screen 500 and the detailed calculation result screen 510.

Although the surface-treatment job history information 610 includes basic information 610a, surface treatment setting information 610b, output information 610c, operation information 610d, and waste information 610e in the present embodiment, this is not limited. The basic information 610a includes, for example, a job ID for identifying a job and the like as with the basic information 600a. The surface treatment setting information 610b includes, for example, the number of sides and the number of copies of sheets on which treatment such as varnishing, foil stamping, filming, or laminating is performed.

The output information 610c relates to an amount of material used in a process until a secondary product is output by the surface treatment apparatus 102. Specifically, the output information 610c includes the total number of pages that have been treated, the amount of varnish consumed when the treatment is varnishing, the area of the foil-stamped sheet when the treatment is foil-stamping, or the like. The varnish consumption and the area of the sheet are processed as a treatment material consumption.

The operation information 610d is an amount of power used in a process until a secondary product is output by the surface treatment apparatus 102, that is, the power consumption consumed from a treatment start time to a treatment end time. The waste information 610e relates to an amount of waste material generated in a process until a secondary product is output by the surface treatment apparatus 102. The waste material is not particularly limited, and includes a sheet that is treated in failure, a sheet for checking treatment, and a sheet that has not been conveyed to the cutting/bookbinding apparatus 103. The number of these sheets are processed as the output number of non-products. Other waste material includes varnish used in treatment of a sheet that has become waste and excess thereof. The amount of varnish and the excess thereof are processed as a treatment material amount of non-products.

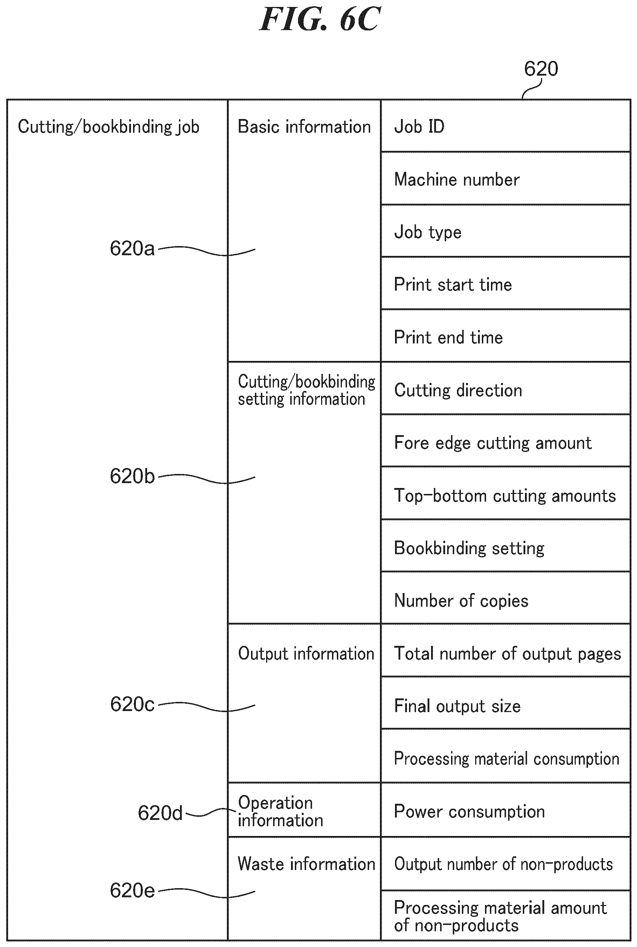

FIG. 6C is a view illustrating cutting/bookbinding job history information 620 required for calculating a CO2 emission in the cutting/bookbinding process among the postprocesses. The cutting/bookbinding job history information (second execution history) 620 illustrated in FIG. 6C is stored in the HDD of the environmental load calculation server 104. The cutting/bookbinding job history information 620 is used as an information included in the job history screen 500 and the detailed calculation result screen 510. Although the cutting/bookbinding job history information 620 includes basic information 620a, cutting/bookbinding setting information 620b, output information 620c, operation information 620d, and waste information 620e in the present embodiment, this is not limited.

The basic information 620a includes, for example, a job ID for identifying a job and the like as with the basic information 600a. The cutting/bookbinding setting information 620b includes, for example, a cutting direction to a sheet, a fore edge cutting amount and top-bottom cutting amounts from sheet edges to designate cutting start positions, a bookbinding setting indicating whether to execute bookbinding and a bookbinding method, and the number of bookbinding copies.

The output information 620c relates to an amount of material used in a process until a secondary product is output by the cutting/bookbinding apparatus 103. Specifically, the output information 620c includes the total number of pages that have been processed, the final size after cutting, and the consumption of processing material used in bookbinding. For example, since saddle stitching binds sheets at two places with wires, two wires are the processing materials. In a case of perfect binding, glue is the processing material.

The operation information 620d is an amount of power used in a process until a secondary product is output by the cutting/bookbinding apparatus 103, that is, a power consumption consumed from a processing start time to a processing end time. The waste information 620e relates to an amount of waste generated in a process until a secondary product is output by the cutting/bookbinding apparatus 103. The waste is not particularly limited and includes a cut piece of a sheet (a surplus of a secondary product) generated by processing (cutting), and a sheet that is processed in failure. The number of such sheets is processed as the output number of non-products. Other waste material includes varnish used in treatment of a sheet that has become waste and excess thereof. The amount of varnish and the excess thereof are processed as a processing material amount of non-products.

The basic information 610a to the waste information 610e and the basic information 620a to the waste information 620e described above relate to the processes until the secondary products are output by the postprocessing apparatus. Hereinafter, the information may be referred to as "second information".

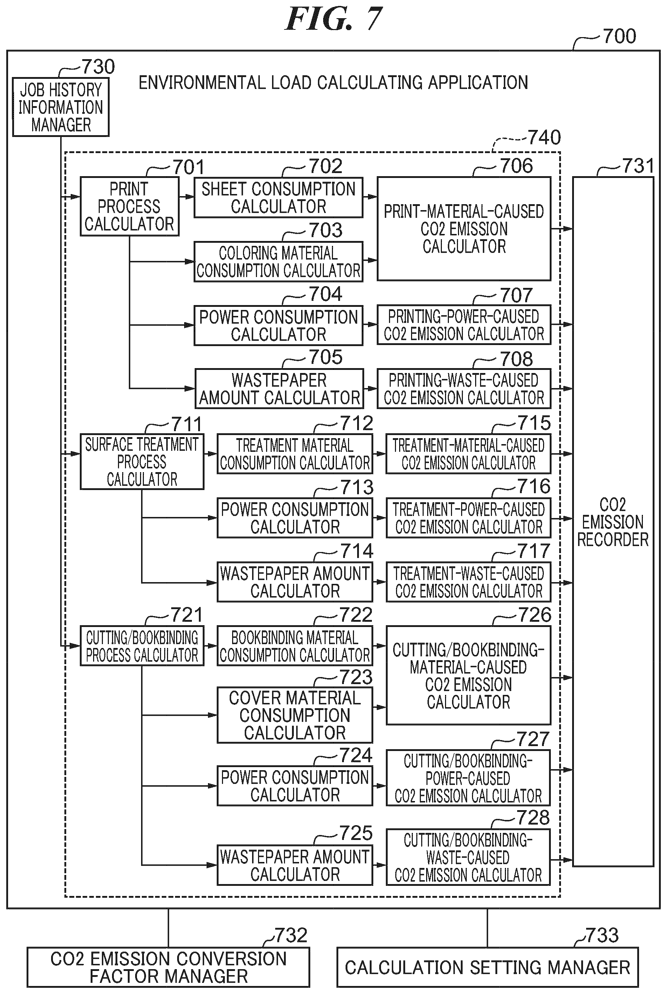

FIG. 7 is a block diagram illustrating an example of a software configuration of the environmental load calculation server 104. As illustrated in FIG. 7, the environmental load calculation server 104 has an environmental load calculating application 700. The environmental load calculating application 700 is software to execute a process of calculating an environmental load and is stored in the **HDD** 493. The CPU 491 reads the environmental load calculating application 700 from the **HDD** 493 to the memory 492 and executes the application 700.

The environmental load calculating application 700 includes a job history information manager 730, a print process calculator 701, a surface treatment process calculator 711, and a cutting/bookbinding process calculator 721. In addition, the environmental load calculating application 700 includes a sheet consumption calculator 702, a coloring material consumption calculator 703, power consumption calculators 704, 713 and 724, wastepaper amount calculators 705, 714, and 725, a print-material -caused CO2 emission calculator 706, a printing-power-caused CO2 emission calculator 707, a printing-waste-caused CO2 emission calculator 708, a treatment material consumption calculator 712, a treatment-material-caused CO2 emission calculator 715, a treatment-power-caused CO2 emission calculator 716, a treatment-waste-caused CO2 emission calculator 717, and a bookbinding material consumption calculator 722, a cover material consumption calculator 723, a cutting/bookbinding-material-caused CO2 emission calculator 726, a cutting/bookbinding-power-caused CO2 emission calculator 727, and a cutting/bookbinding-waste-caused CO2 emission calculator 728. The environmental load calculating application 700 further includes a CO2 emission recorder 731, a CO2 emission conversion factor manager 732, and a calculation setting manager 733.

When the CO2 emission calculation button 505 is pressed in a state where a desired checkbox of the column 502 on the job history screen 500 is selected, the job history information manager 730 is notified of the job ID of the job corresponding to the selected checkbox of the column 502.

The job history information manager 730 obtains the job history information (the first information and the second information) based on the job ID (an obtaining step). As described above, in the present embodiment, the job history information manager 730 functions as an obtaining unit that obtains the job history information.

In addition, the job history information manager 730 calls any one of the print process calculator 701, the surface treatment process calculator 711, and the cutting/bookbinding process calculator 721 in accordance with the job type of the job included in the job history information. For example, when the job type is the print, the job history information manager 730 calls the print process calculator 701. When the job type is the surface treatment, the job history information manager 730 calls the surface treatment process calculator 711. When the job type is the cutting/bookbinding, the job history information manager 730 calls the cutting/bookbinding process calculator 721.

The print process calculator 701 executes a process to calculate a CO2 emission from the print job history. The print process calculator 701 calls the sheet consumption calculator 702, the coloring material consumption calculator 703, the power consumption calculator 704, and the wastepaper amount calculator 705 on the basis of the print job history information.

The sheet consumption calculator 702 reads the number of sheets for each medium of the output information 600c in the print job history information 600, and stores the number of sheets in association with the medium ID in the memory 492 as the sheet consumption. The coloring material consumption calculator 703 reads a coloring material consumption (toner consumption) of the output information 600c in the print job history information 600 and stores the coloring material consumption in the memory 492.

The power consumption calculator 704 reads the power consumption of the output information 600c in the print job history information 600 and stores the power consumption in the memory 492. The wastepaper amount calculator 705 reads the output number of non-products of the output information 600c in the print job history information 600, and stores the output number of non-products in association with the medium ID in the memory 492 as the wastepaper amount.

A CO2 emission conversion factor (predetermined coefficient) for each medium corresponding to each medium ID is stored in the HDD 493 in advance. The print-material-caused CO2 emission calculator 706 reads the CO2 emission conversion factor from the HDD 493 and multiplies the sheet consumption in the memory 492 by the CO2 emission conversion factor. As a result, the CO2 emission corresponding to the sheet consumption is calculated. The calculation result is stored in the memory 492. In addition, a CO2 emission conversion factor for each coloring material in the printer 105 is stored in the HDD 493 in advance.

The print-material-caused CO2 emission calculator 706 reads the CO2 emission conversion factor from the HDD 493 and multiplies the coloring material consumption in the memory 492 by the CO2 emission conversion factor. Accordingly, the CO2 emission corresponding to the coloring material consumption is calculated. The calculation result is stored in the memory 492. In this manner, the print-material-caused CO2 emission calculator 706 can calculate the CO2 emission of a raw material stage on the basis of the sheet consumption and the coloring material consumption stored in the memory 492.

In addition, a CO2 emission conversion factor corresponding to the power consumption is stored in the HDD 493 in advance. Note that the CO2 emission conversion factor may be a coefficient obtained by simulation or experiment, or may be a coefficient provided from an electric power company, for example. The printing-power-caused CO2 emission calculator 707 calls the CO2 emission conversion factor from the HDD 493 and multiplies the power consumption in the memory 492 by the CO2 emission conversion factor. As a result, a CO2 emission corresponding to the power consumption is calculated. The calculation result is stored in the memory 492.

In addition, a CO2 emission conversion factor related to waste of each medium corresponding to the medium ID is stored in the HDD 493 in advance. The printing-waste-caused CO2 emission calculator 708 calls the CO2 emission conversion factor from the HDD 493 and multiplies the output number of non-products (sheet consumption) in the memory 492 by the CO2 emission conversion factor. Thereby, a CO2 emission corresponding to the output number of non-products is calculated. The calculation result is stored in the memory 492. In the calculation of the CO2 emission in a waste stage, a recycling rate of the waste may be included in the calculation conditions. The recycle ratio can be set by, for example, the calculation setting button 503.

As described above, the environmental load calculating application 700 (environmental load calculation server 104) can calculate the CO2 emission emitted by the image forming apparatus 101 on the basis of the first information (a first calculation step). In the environmental load calculating application 700, the calculator group from the print process calculator 701 to the printing-waste-caused CO2 emission calculator 708 functions as a first operation unit 740 that executes the first operation step.

The surface treatment process calculator 711 calls the treatment material consumption calculator 712, the power consumption calculator 713, and the wastepaper amount calculator 714 on the basis of the surface-treatment job history information. The treatment material consumption calculator 712 reads the processing material consumption of the output information 610c in the surface-treatment job history information 610 and stores the treatment material consumption in the memory 492. The power consumption calculator 713 reads the power consumption of the operation information 610d in the surface-treatment job history information 610 and stores the power consumption in the memory 492. The wastepaper amount calculator 714 reads the output number of non-products of the waste information 610e in the surface-treatment job history information 610, and stores the output number of non-products in the memory 492.

In the HDD493, a CO2 emission conversion factor corresponding to the treatment material consumption in the surface treatment apparatus 102 is stored in advance. The treatment-material-caused CO2 emission calculator 715 calls this CO2 emission conversion factor from the HDD 493 and multiplies it by the treatment material consumption in the memory 492. As a result, a CO2 emission corresponding to the treatment material consumption is calculated. The calculation result is stored in the memory 492.

In addition, a CO2 emission conversion factor corresponding to the power consumption in the surface treatment apparatus 102 is stored in the HDD 493 in advance. The treatment-power-caused CO2 emission calculator 716 calls the CO2 emission conversion factor from the HDD 493 and multiplies the power consumption in the memory 492 by the CO2 emission conversion factor. As a result, a CO2 emission corresponding to the power consumption is calculated. The calculation result is stored in the memory 492.

In addition, a CO2 emission conversion factor related to waste of each medium corresponding to the medium ID is stored in the HDD 493 in advance. The treatment-waste-caused CO2 emission calculator 717 calls the CO2 emission conversion factor from the HDD 493 and multiplies the output number of non-products in the memory 492 by the CO2 emission conversion factor. Thereby, a CO2 emission corresponding to the output number of non-products is calculated. The calculation result is stored in the memory 492. In the calculation of the CO2 emission in a waste stage, a recycling rate of the waste may be included in the calculation conditions as with the calculation by the printing-waste-caused CO2 emission calculator 708.

The cutting/bookbinding process calculator 721 calls the bookbinding material consumption calculator 722, the cover material consumption calculator 723, the power consumption calculator 724, and the wastepaper amount calculator 725 on the basis of the cutting/bookbinding job history information. The bookbinding material consumption calculator 722 reads the processing material consumption of the output information 620c in the cutting/bookbinding job history information 620, and stores the processing material consumption in the memory 492.

The cover material consumption calculator 723 reads the cover material consumption of the output information 620c in the cutting/bookbinding job history information 620, and stores the cover material consumption in the memory 492. The cover material consumption is the number of sheets constituting a cover in bookbinding. The power consumption calculator 724 reads the operation information 620d in the cutting/bookbinding job history information 620, and stores it in the memory 492. The wastepaper amount calculator 725 calls the output number of non-products of the waste information 620e in the cutting/bookbinding job history information 620, and stores it in the memory 492.

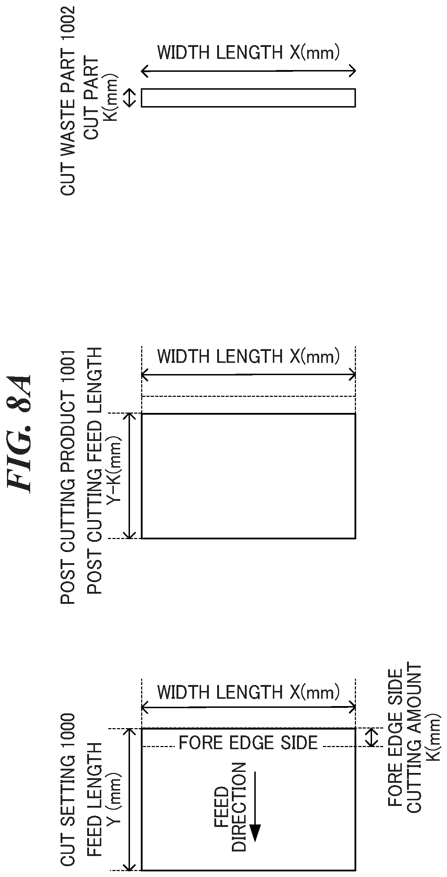

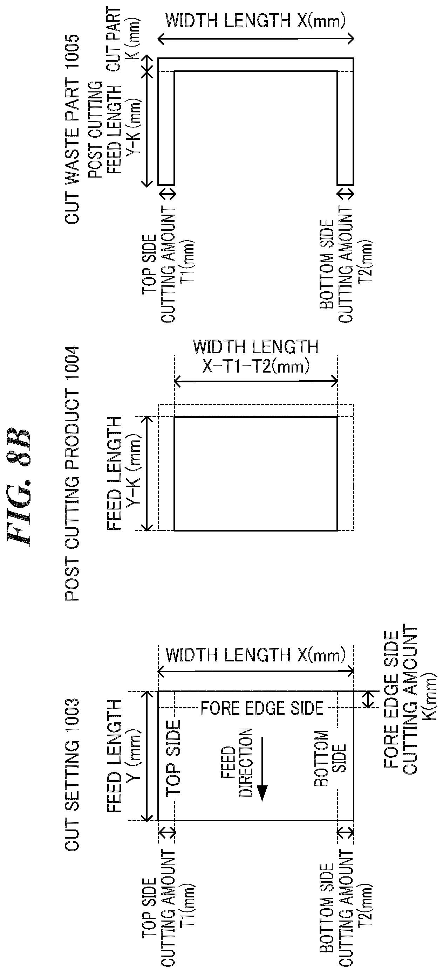

Here, cutting of a sheet and a waste part (surplus part) generated by the cutting will be described with reference to FIG. 8A and FIG. 8B. FIG. 8A is a view to describe a relationship between cutting of a sheet on a fore edge side and a waste part (surplus part) generated by the cutting. In a cut setting 1000 shown in FIG. 8A, a fore edge side cutting amount K (unit: mm) is set for a sheet having a width length X (unit: mm) and a feed length Y (unit: mm). Then, a post cutting sheet 1001 is obtained by cutting the sheet at a position of the fore edge side cutting amount K from the edge of the fore edge side. This cutting also produces a cut waste part 1002. The cut waste part 1002 has an area found by (K · X). Then, the total cutting weight for each medium is calculated by multiplying the area by the basis weight of the medium and the output number.

FIG. 8B is a view illustrating a case where three sides are cut. In the case of three side cutting, a top side and a bottom side of the sheet are cut after cutting the fore edge side of the sheet. In a cutting setting 1003 shown in FIG. 8B, the cutting of the fore edge side is performed at the position of the fore edge side cutting amount K from the edge of the fore edge side, similarly to FIG. 8A. The cutting on the top side is performed at a position of a top side cutting amount T1 (unit: mm) from the sheet edge of the top side. The cutting on the bottom side is performed at a position of a bottom side cutting amount T2 (unit: mm) from the sheet edge of the bottom side. Then, a post cutting sheet 1004 is obtained by cutting the fore edge side, the top side, and the bottom side. This cutting also produces a cut waste part 1005. The waste part 1005 has an area found by (K · X + T1 · (Y - K) + T2 · (Y - K)). Then, the total cutting weight of the waste part 1005 is calculated by multiplying the area by the basis weight of the medium and the output number.

In addition, when a plurality of media are used, the total cutting weights are calculated for the number of media. Then, as a calculation condition for calculating the CO2 emissions corresponding to the total cutting weight of the waste part 1002 or the waste part 1005, the CO2 emission conversion factor that corresponds to the type of the sheet (for example, wood-free paper, coated paper, or recycled paper) used as a raw material of the printed matter is used.

In the HDD 493, a CO2 emission conversion factor corresponding to the processing material consumption used in the cutting/bookbinding apparatus 103 is stored in advance. The cutting/bookbinding-material-caused CO2 emission calculator 726 calls this CO2 emission conversion factor from the HDD 493 and multiplies it by the processing material consumption in the memory 492. As a result, a CO2 emission corresponding to the processing material consumption is calculated. The calculation result is stored in the memory 492.

In addition, a CO2 emission conversion factor corresponding to the power consumption in the cutting/bookbinding apparatus 103 is stored in the HDD 493 in advance. The cutting/bookbinding-power-caused CO2 emission calculator 727 calls the CO2 emission conversion factor from the HDD 493 and multiplies the power consumption in the memory 492 by the CO2 emission conversion factor. As a result, a CO2 emission corresponding to the power consumption is calculated. The calculation result is stored in the memory 492.

In addition, a CO2 emission conversion factor related to waste of each medium corresponding to the medium ID is stored in the HDD 493 in advance. The cutting/bookbinding-waste-caused CO2 emission calculator 728 calls the CO2 emission conversion factor from the HDD 493 and multiplies the output number of non-products in the memory 492 by the CO2 emission conversion factor. Thereby, a CO2 emission corresponding to the output number of non-products is calculated. The calculation result is stored in the memory 492. In the calculation of the CO2 emission in the waste stage, the recycling rate of the waste may be included in the calculation conditions as with the calculations by the printing-waste-caused CO2 emission calculator 708 and the treatment-waste-caused CO2 emission calculator 717.

As described above, the environmental load calculating application 700 (environmental load calculation server 104) can calculate the CO2 emission emitted by the post-processing apparatus on the basis of the second information (the first calculation step). In the environmental load calculating application 700, the calculator group from the surface treatment process calculator 711 to the treatment-waste-caused CO2 emission calculator 717 and the calculator group from the cutting/bookbinding process calculator 721 to the cutting/bookbinding-waste-caused CO2 emission calculator 728 function as the first operation unit 740 that executes the first operation step.

Although the calculator group from the print process calculator 701 to the printing-waste-caused CO2 emission calculator 708, the calculator group from the surface treatment process calculator 711 to the treatment-waste-caused CO2 emission calculator 717, and the calculator group from the cutting/bookbinding process calculator 721 to the cutting/bookbinding-waste-caused CO2 emission calculator 728 collectively constitute one operation unit in the present embodiment, this is not limited. For example, the calculator group from the print process calculator 701 to the printing-waste-caused CO2 emission calculator 708, the calculator group from the surface treatment process calculator 711 to the treatment-waste-caused CO2 emission calculator 717, and the calculator group from the cutting/bookbinding process calculator 721 to the cutting/bookbinding-waste-caused CO2 emission calculator 728 may constitute respective calculation units.

The CO2 emission recorder 731 stores the calculation results of the print-material-caused CO2 emission calculator 706, the printing-power-caused CO2 emission calculator 707, and the printing-waste-caused CO2 emission calculator 708, that is, the CO2 emission in the image forming apparatus 101, in the HDD 493. Further, the CO2 emission recorder 731 stores the calculation results of the treatment-material-caused CO2 emission calculator 715, the treatment-power-caused CO2 emission calculator 716, and the treatment-waste-caused CO2 emission calculator 717, that is, the CO2 emission of the surface treatment apparatus 102, in the HDD 493.

Further, the CO2 emission recorder 731 stores the calculation results of the cutting/bookbinding-material-caused CO2 emission calculator 726, the cutting/bookbinding-power-caused CO2 emission calculator 727, and the cutting/bookbinding-waste-caused CO2 emission calculator 728, that is, the CO2 emission of the cutting/bookbinding apparatus 103, in the HDD 493. The calculation results are stored for each process and each stage. In addition, when the calculation results in the same process or the same stage are stored, a value obtained by adding up the calculation results stored in the respective processes is also stored. Each stored calculation result is associated with a predetermined ID.

Further, the CO2 emission recorder 731 calculates the total CO2 emission on the basis of the respective calculation results (a second calculation step). Specifically, the CO2 emission recorder 731 calculates the total CO2 emission by adding up the CO2 emission of the image forming apparatus 101, the CO2 emission of the surface treatment apparatus 102, and the CO2 emission of the cutting/bookbinding apparatus 103. As described above, in the present embodiment, the CO2 emission recorder 731 has a function as a second calculation unit that calculates the total CO2 emission.

The CO2 emission conversion factor manager 732 manages a factor to convert a CO2 emission. The CO2 emission conversion factor manager 732 holds conversion information that associates a conversion item and a conversion factor. The conversion information is also stored in the HDD 493, and is read to the memory 492 when each CO2 emission described above is calculated. The conversion information can be updated.

The calculation setting manager 733 manages a set value used in calculating a CO2 emission. The calculation setting manager 733 also manages a determination criterion (calculation rule) used in determining whether to store the total CO2 emission in the HDD 493. The determination criterion is not particularly limited, and may be, for example, whether there is a setting on a setting screen displayed by pressing the calculation setting button 503 on the job history screen 500. The determination criterion can be updated. The calculation setting manager 733 accepts reading of a set value from each module constituting the environmental load calculating application 700 and transfers the set value to each module.

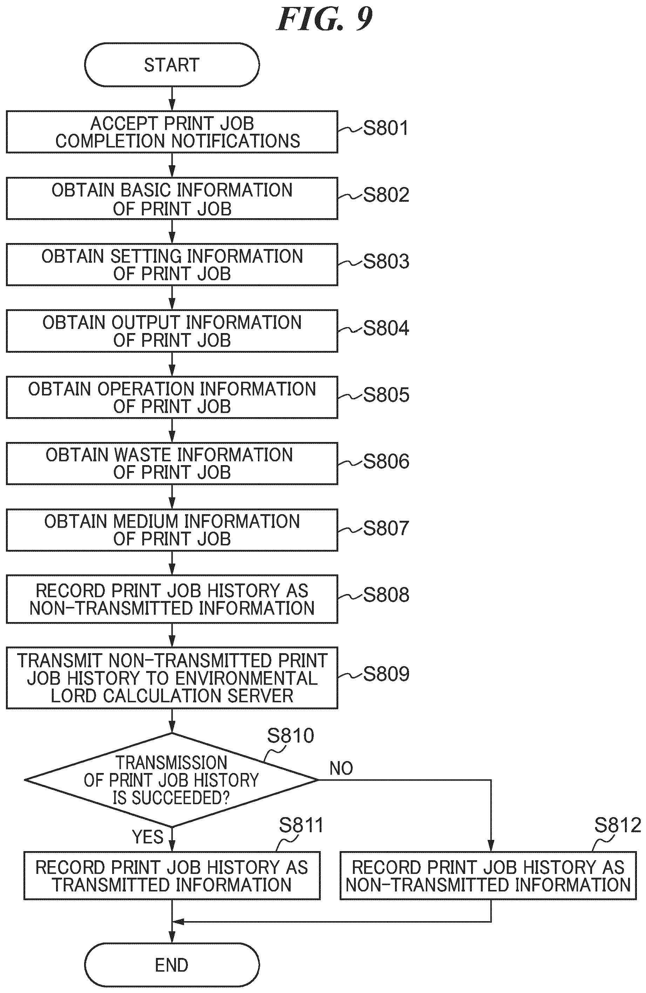

FIG. 9 is a flowchart illustrating a process executed by the image forming apparatus. This process is executed when the image forming apparatus 101 completes a job and transmits information related to the calculation of a CO2 emission to the environmental load calculation server 104. A program based on the flowchart illustrated in FIG. 9 is stored in the HDD 404 of the printer 105. The CPU 405 of the printer 105 loads the program from the HDD 404 onto the memory 406 and executes the program.

As illustrated in FIG. 9, in a step S801, the CPU 405 accepts print job completion notifications from the sheet feed unit 413 of the printer 105, the large-capacity stacker 108, and the finisher 109. Accordingly, the CPU 405 determines the completion of the print job. After the execution of the step S801, the process proceeds to a step S802.

In the step S802, the CPU 405 obtain the basic information 600a (see FIG. 6A) of the print job determined to be completed in the step S801 from the HDD 404 and stores the basic information 600a in the memory 406. After the execution of the step S802, the process proceeds to a step S803.

In the step S803, the CPU 405 obtains the print setting information 600b (see FIG. 6A) of the print job determined to be completed in the step S801 from the HDD 404 and stores the print setting information 600b in the memory 406. After the execution of the step S803, the process proceeds to a step S804.

In the step S804, the CPU 405 obtains the output information 600c (see FIG. 6A) of the print job determined to be completed in the step S801 from the HDD 404 and stores the output information 600c in the memory 406. After the execution of the step S804, the process proceeds to a step S805.

In the step S805, the CPU 405 obtains the operation information 600d (see FIG. 6A) of the print job determined to be completed in the step S801 from the HDD 404 and stores the operation information 600d in the memory 406. After the execution of the step S805, the process proceeds to a step S806.

In the step S806, the CPU 405 obtains the waste information 600e (see FIG. 6A) of the print job determined to be completed in the step S801 from the HDD 404 and stores the waste information 600e in the memory 406. After the execution of the step S806, the process proceeds to a step S807.

In the step S807, the CPU 405 obtains the medium information 600f (see FIG. 6A) of the print job determined to be completed in the step S801 from the HDD 404 and stores the medium information 600f in the memory 406. After the execution of the step S807, the process proceeds to a step S808.

In the step S808, the CPU 405 stores (records) the information stored in the memory 406 in the steps S802 to S807 in the HDD 404 as non-transmitted information related to the corresponding job ID. After the execution of the step S808, the process proceeds to a step S809.

In the step S809, the CPU 405 transmits the print job history that has been saved as the non-transmitted information in the HDD 404 in the step S808 to the environmental load calculation server 104 via the LAN I/F 402. After the execution of the step S809, the process proceeds to a step S810.

In the step S810, the CPU 405 determines whether the transmission of the print job history in the step S809 is succeeded. As a result of the determination in the step S810, when it is determined that the transmission is succeeded, the process proceeds to a step S811. In this case, the print job history transmitted from the printer 105 is stored in the HDD 493 of the environmental load calculation server 104. On the other hand, as a result of the determination in the step S810, when it is determined that the transmission is not succeeded, that is, the transmission failed, the process proceeds to a step S812.

In the step S811, the CPU 405 stores the print job history transmitted in the step S809 as transmitted information in the HDD 404. After the execution of the step S811, the process ends.

In the step S812, the CPU 405 stores the print job history transmitted in the step S809 as non-transmitted information in the HDD 404. After the execution of the step S812, the process ends.

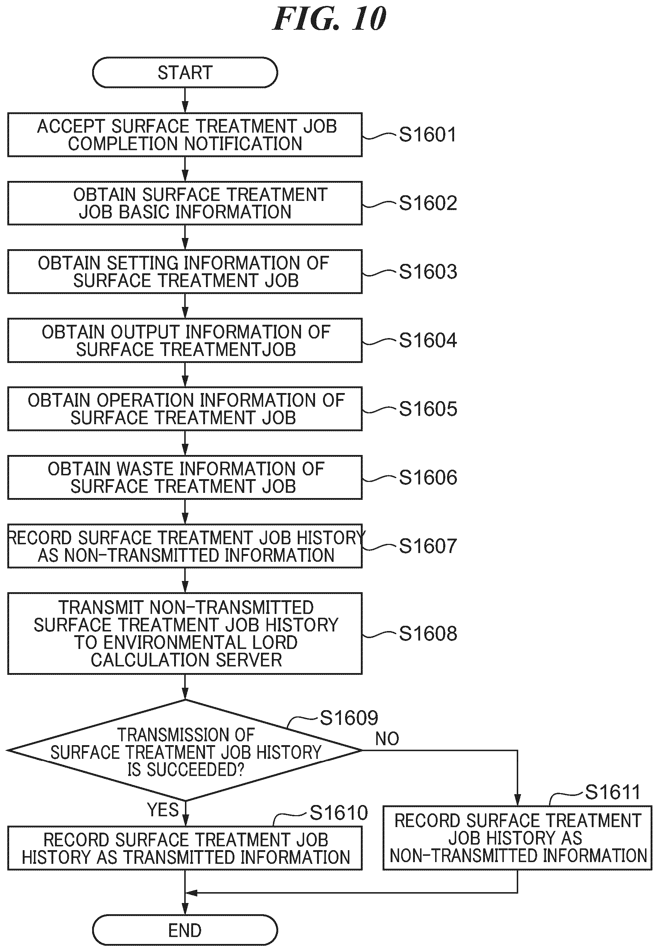

FIG. 10 is a flowchart illustrating a process executed by the postprocessing apparatus. This process is executed when the postprocessing apparatus completes a job and transmits information related to the calculation of a CO2 emission to the environmental load calculation server 104. As described above, in the present embodiment, the surface treatment apparatus 102 and the cutting/bookbinding apparatus 103 are included as the postprocessing apparatuses. Here, a flowchart illustrating the process executed by the surface treatment apparatus 102 will be described as a representative example.

The program based on the flowchart illustrated in FIG. 10 is stored in the HDD 473 of the surface treatment apparatus 102. The CPU 405 of the surface treatment apparatus 102 loads the program from the HDD 473 onto the memory 472 and executes the program. As illustrated in FIG. 10, in a step S1601, the CPU 471 accepts a surface treatment job completion notification from the surface treatment unit 478 of the surface treatment apparatus 102. The CPU 471 thereby determines completion of the surface treatment job. After the execution of the step S1601, the process proceeds to a step S1602.

In the step S1602, the CPU 471 obtains the basic information 610a (see FIG. 6B) of the surface treatment job determined to be completed in the step 1601 from the HDD 473 and stores the basic information 610a in the memory 472. After the execution of the step S1602, the process proceeds to a step S1603.

In step S1603, the CPU 471 obtains the surface treatment setting information 610b (see FIG. 6B) of the surface treatment job determined to be completed in the step S1601 from the HDD 473 and stores the setting information 610b in the memory 472. After the execution of the step S1603, the process proceeds to a step S1604.

In step S1604, the CPU 471 obtains the output information 610c (see FIG. 6B) of the surface treatment job determined to be completed in the step S1601 from the HDD 473 and stores the output information 610c in the memory 472. After the execution of the step S1604, the process proceeds to a step S1605.

In step S1605, the CPU 471 obtains the operation information 610d (see FIG. 6B) of the surface treatment job determined to be completed in the step S1601 from the HDD 473 and stores the output information 610d in the memory 472. After the execution of the step S1605, the process proceeds to a step S1606.

In step S1606, the CPU 471 obtains the waste information 610e (see FIG. 6B) of the surface treatment job determined to be completed in the step S1601 from the HDD 473 and stores the waste information 610e in the memory 472. After the execution of the step S1606, the process proceeds to a step S1607.

In the step S1607, the CPU 471 stores the information stored in the memory 472 in the steps S1602 to S1606 in the HDD 473 as non-transmitted information related to the corresponding job ID. After the execution of the step S1607, the process proceeds to a step S1608.

In the step S1608, the CPU 471 transmits the non-transmitted surface treatment job history stored in the HDD 473 in the step S1607 to the environmental load calculation server 104 via the LAN I/F 474. After the execution of the step S1608, the process proceeds to a step S1609.

In the step S1609, the CPU 471 determines whether the transmission of the surface treatment job history in the step S1608 is succeeded. As a result of the determination in the step S1609, when it is determined that the transmission is succeeded, the process proceeds to a step S1610. In this case, the surface treatment job history transmitted from the surface treatment apparatus 102 is stored in the HDD 493 of the environmental load calculation server 104. On the other hand, as a result of the determination in the step S1609, when it is determined that the transmission is not succeeded, that is, the transmission failed, the process proceeds to a step S1611.

In the step S1610, the CPU 471 stores the surface treatment job history transmitted in the step S1608 as transmitted information in the HDD 473. After the execution of the step S1610, the process ends.

In the step S1611, the CPU 471 stores the surface treatment job history transmitted in step S1608 as non-transmitted information in the HDD 473. After the execution of the step S1611, the process ends.

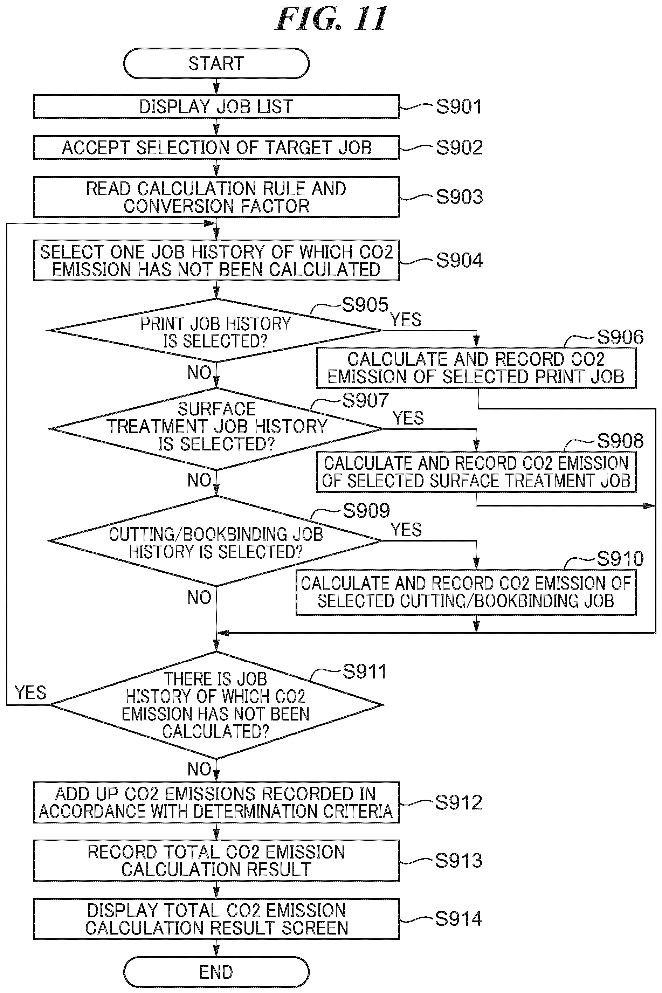

FIG. 11 is a flowchart illustrating a process executed by the environmental load calculation server 104 to calculate a CO2 emission. A program based on the flowchart illustrated in FIG. 11 is stored in the HDD 493 of the environmental load calculation server 104. The CPU 491 of the environmental load calculation server 104 loads the program from the HDD 493 onto the memory 492 and executes the program. As illustrated in FIG. 11, in a step S901, the CPU 491 generates the job history screen 500 (see FIG. 5A) on the basis of the information transmitted from surface treatment apparatus 102 and stored in the HDD 493, and displays the job history screen 500 on the display unit 496. After the execution of the step S901, the process proceeds to a step S902.

In the step S902, the CPU 491 waits for a user operation for the checkboxes in the column 502 and the CO2 emission calculation button 505 on the job history screen 500 displayed on the display unit 496 in the step S901. When the checkboxes in the column 502 are selected via the operation unit 495 and the CO2 emission calculation button 505 is pressed, the CPU 491 stores the job IDs of the job history information corresponding to the selected checkboxes in the column 502 in the memory 492 as the calculation target job IDs. After the execution of the step S902, the process proceeds to a step S903.

In the step S903, the CPU 491 reads the determination criterion and the conversion factors necessary for conversion of the CO2 emission stored in the HDD 493, and develops the determination criterion and the conversion factor in the memory 492. The conversion factors are stored in the HDD 493 for the print process, the surface treatment process, and the cutting/bookbinding process, respectively. After the execution of the step S903, the process proceeds to a step S904.

In the step S904, the CPU 491 selects one job history (item) of which a CO2 emission is not calculated from among the calculation target job IDs stored in the memory 492 in the step S902 and reads the job history (item) onto the memory 492. After the execution of the step S904, the process proceeds to a step S905.

In the step S905, the CPU 491 determines whether the job history read onto the memory 492 in the step S904 is a job history of the print process, that is, whether a print job history is selected. As a result of the determination in the step S905, when it is determined that the print job history is selected, the process proceeds to a step S906. On the other hand, as a result of the determination in the step S905, when it is determined that the print job history is not selected, the process proceeds to a step S907.

In the step S906, the CPU 491 calls the print process calculator 701 of the environmental load calculating application 700 on the basis of the job history of the calculation target job selected in the step S904. Then, the CPU 491 executes the calculation process for calculating the CO2 emission in the print process by using the print process calculator 701 and the calculator group from the sheet consumption calculator 702 to the printing-waste-caused CO2 emission calculator 708. The calculation result is stored in the memory 492. After the execution of the step S906, the process proceeds to a step S911.

In the step S907, the CPU 491 determines whether the job history read onto the memory 492 in the step S904 is a job history of the surface treatment process, that is, whether a surface treatment job history is selected. As a result of the determination in the step S907, when it is determined that the surface treatment job history is selected, the process proceeds to a step S908. On the other hand, as a result of the determination in the step S905, when it is determined that the surface treatment job history is not selected, the process proceeds to a step S909.

In the step S908, the CPU 491 calls the surface treatment process calculator 711 of the environmental load calculating application 700 on the basis of the job history of the calculation target job selected in the step S904. Then, the CPU 491 executes the calculation process for calculating the CO2 emission in the surface treatment process by using the surface treatment process calculator 711 and the calculator group from the treatment material consumption calculator 712 to the treatment-waste-caused CO2 emission calculator 717. The calculation result is stored in the memory 492. After the execution of the step S908, the process proceeds to a step S911.

In the step S909, the CPU 491 determines whether the job history read onto the memory 492 in the step S904 is a job history of the cutting/bookbinding process, that is, whether the cutting/bookbinding job history is selected. As a result of the determination in the step S909, when it is determined that the cutting/bookbinding job history is selected, the process proceeds to a step S910. On the other hand, as a result of the determination in the step S905, when it is determined that the cutting/bookbinding job history is not selected, the process proceeds to the step S911.

In the step S910, the CPU 491 calls the cutting/bookbinding process calculator 721 of the environmental load calculating application 700 on the basis of the job history of the calculation target job selected in the step S904. Then, the CPU 491 executes the calculation process for calculating the CO2 emission in the cutting/bookbinding process by using the cutting/bookbinding process calculator 721 and the calculator group from the bookbinding material consumption calculator 722 to the cutting/bookbinding-waste-caused CO2 emission calculator 728. The calculation result is stored in the memory 492. After the execution of the step S910, the process proceeds to the step S911.

In the step S911, the CPU 491 determines whether there is a job history of which a CO2 emission has not been calculated among the calculation target job IDs stored in the memory 492 in the step S902. As a result of the determination in the step S911, when it is determined that there is a job history of which a CO2 emission has not been calculated, the process returns to the step S904, and the subsequent steps are executed in order. On the other hand, as a result of the determination in the step S911, when it is determined that there is no job history of which a CO2 emission has not been calculated, the process proceeds to a step S912.

In the step S912, the CPU 491 reads the calculation results stored in the memory 492 up to the step S911 in accordance with the determination criteria managed by the calculation setting manager 733. Also, the CPU 491 calls the CO2 emission recorder 731 of the environmental load calculating application 700. The CPU 491 calculates the total CO2 emission by summing up the read calculation results using the CO2 emission recorder 731. After the execution of the step S912, the process proceeds to a step S913.

In the step S913, the CPU 491 stores the total CO2 emission stored in the memory 492 in the step S912 in the HDD 493 as a CO2 emission calculation history. After the execution of the step S913, the process proceeds to a step S914.

In the step S914, the CPU 491 generates the detailed calculation result screen 510 capable of displaying the CO2 emission calculation history stored in the HDD 493 in the step S913 and displays the generated screen on the display unit 496. The total CO2 emission based on the CO2 emission calculation history is displayed in the total CO2 emission display field 512 on the detailed calculation result screen 510. After the execution of the step S914, the process ends.

As described above, in the information processing system 1, the environmental load calculation server 104 obtains the first information related to the processes until the primary product is output by the image forming apparatus 101 from the image forming apparatus 101. The environmental load calculation server 104 calculates the CO2 emission emitted by the image forming apparatus 101 on the basis of the first information. The environmental load calculation server 104 obtains the second information related to the processes until the secondary product is output by the postprocessing apparatus from the postprocessing apparatus.

The environmental load calculation server 104 calculates the CO2 emission emitted from the postprocessing apparatus on the basis of the second information. Then, the environmental load calculation server 104 can obtain the total CO2 emission generated until the secondary product is obtained by adding the CO2 emission in the image forming apparatus 101 and the CO2 emission in the postprocessing apparatus.

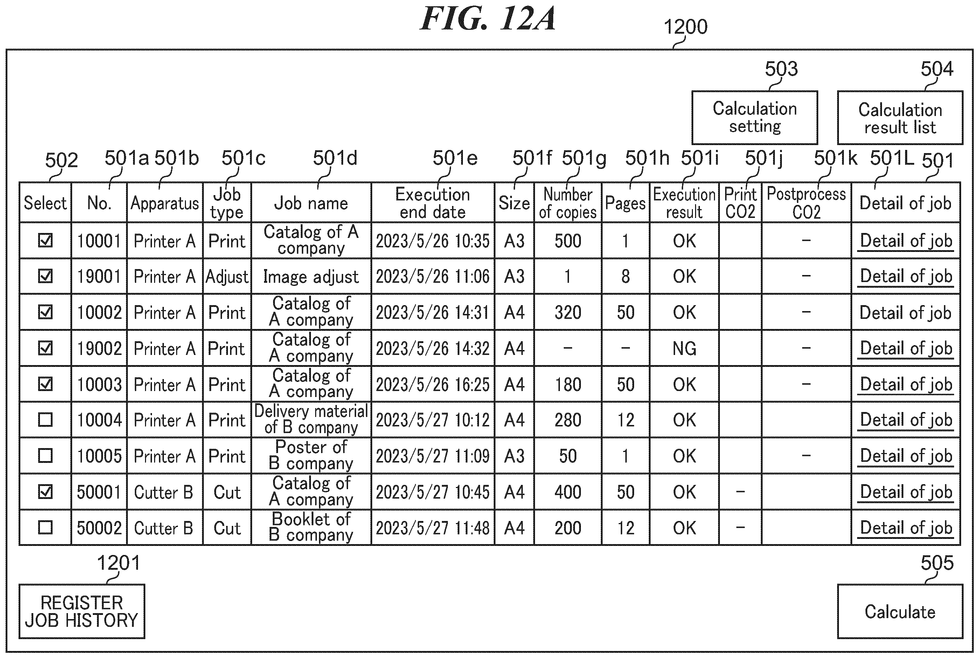

Hereinafter, a second embodiment will be described with reference to FIG. 12A to FIG. 13. Differences from the above-described embodiment will be mainly described, and the description of the same matters will be omitted. For example, there may be a case where the surface treatment apparatus 102 and the cutting/bookbinding apparatus 103 operate without being connected to the network. In this case, in order to calculate the environmental load of the entire product, the environmental load calculation server 1041 needs to separately receive contents of jobs executed by the surface treatment apparatus 102 and the cutting/bookbinding apparatus 103. In the present embodiment, a configuration in which the environmental load calculation server 104 separately receives the contents of the jobs will be described.

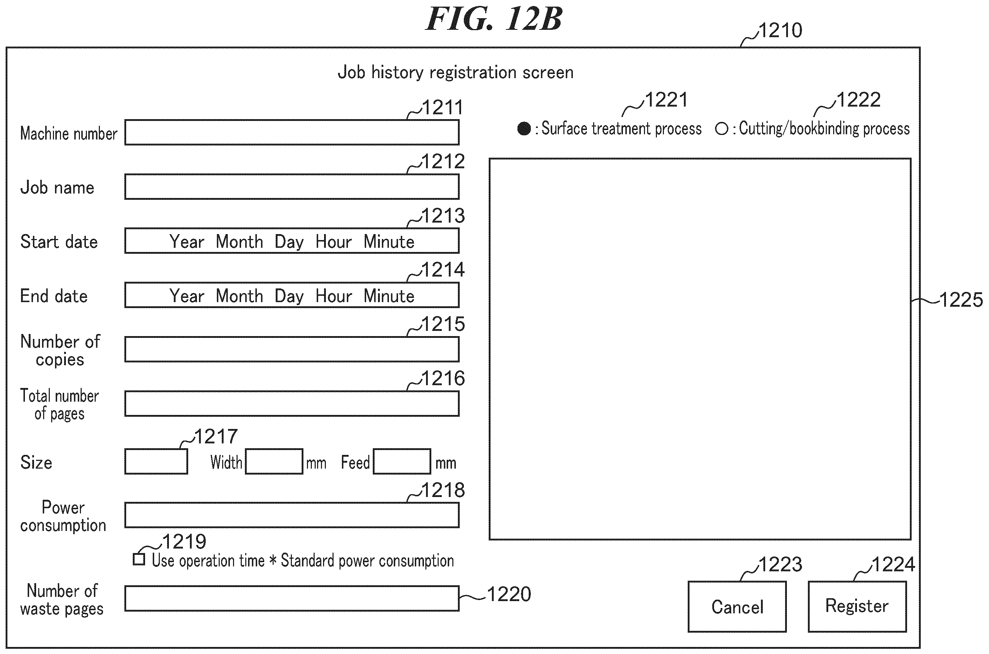

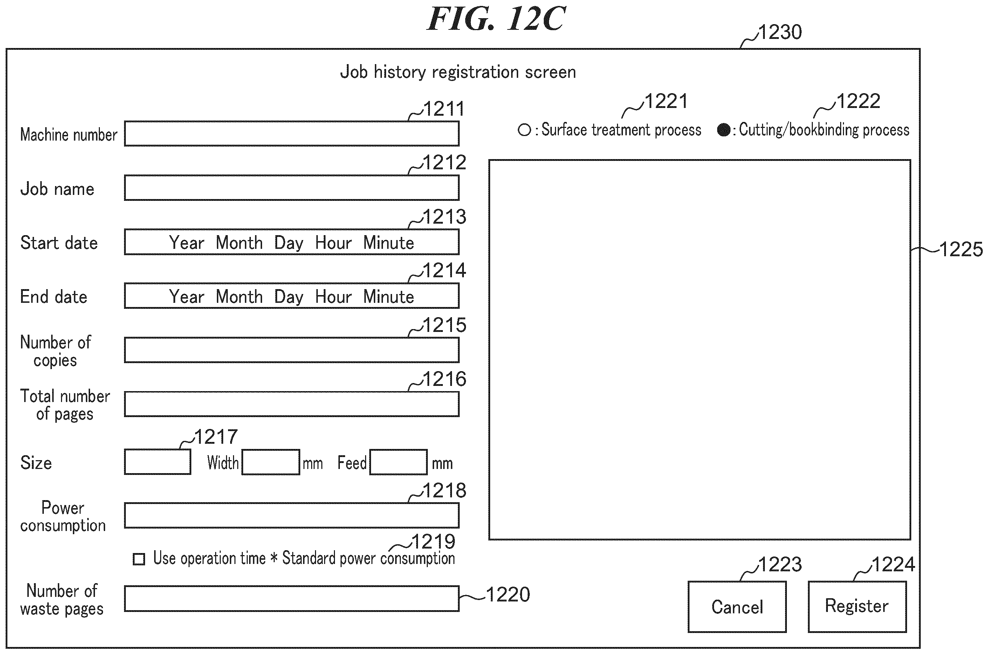

FIG. 12A is a view illustrating an example of a history screen listing jobs that can be a target of the environmental load calculation in the image forming apparatus and the cutting/bookbinding apparatus according to the second embodiment. FIG. 12B is a view illustrating an example of a display screen to which a job history of the surface treatment process is input. FIG. 12C is a view illustrating an example of a display screen on which a job history of the cutting/bookbinding process is input. As shown in FIG. 12A, the job history screen 1200 includes the job history table 501, the calculation setting button 503, the calculation result list button 504, the CO2 emission calculation button 505, and a job history registration button 1201. The job history table 501 includes the column 502 and the columns 501a to 501L as with FIG. 5A. The job history registration button 1201 is used to instruct registration of a job history on the environmental load calculation server 104 separately from the job history received via the network. When the job history registration button 1201 is pressed, a job history registration screen 1210 shown in FIG. 12B or a job history registration screen 1230 shown in FIG. 12C is displayed.

The information corresponding to the surface-treatment job history information 610 (see FIG. 6B) is input on the job history registration screen 1210. The information corresponding to the cutting/bookbinding job history information 620 (see FIG. 6C) is input on the job history registering screen 1230. Note that the operation unit 495 is used for an input operation on the job history registration screen 1210 and the job history registration screen 1230. Since the job history registration screen 1210 and the job history registration screen 1230 have the same screen content, the job history registration screen 1210 will be described as a representative. The job history registration screen 1210 includes fields from a machine number field 1211 to a job history information field 1225.

In the machine number field 1211, identification information of an apparatus that has executed a job to be registered in the job history is input. The identification information may be selected from a target apparatus list stored in the environmental load calculation server 104 or a machine number list registered in advance. A job name field 1212 is used to input a name of a job. A start date field 1213 is used to input start date and time of a job. An end date field 1214 is used to input end date and time of a job. The input date and time may be input as a numerical value or may be input using a date and time input screen on a displayed calendar.

A number-of-copies field 1215 is used to input the number of copies of products processed in a job. A total-number-of-pages field 1216 is used to input the total number of pages obtained by multiplying the number of pages to be processed in one job by the number of copies input in the number-of-copies field 1215. A size field 1217 is used to input a size of a sheet to be processed in a job. A size may be input in accordance with a standard such as A4 or A3, or may be input as a numerical value for each of the width and feed directions. A power consumption field 1218 is used to input a power consumption consumed by executing a job. A standard power consumption use setting checkbox 1219 is used to select whether to use an input value input to the power consumption field 1218 (when not checked) or to use a value obtained by multiplying an operation time by a standard power consumption (when checked). When the standard power consumption use setting checkbox 1219 is checked, the value input to the power consumption field 1218 may be ignored or the input to the power consumption field 1218 may not be accepted.

A number-of-waste-pages field 1220 is used to input the number of pages wasted when a job is executed. One of a surface treatment process selection radio button 1221 or a cutting/bookbinding process selection radio button 1222 is selected. When the surface treatment process selection radio button 1221 is selected (FIG. 12B), the job history information of the surface treatment process is input to the job history information field 1225. When the cutting/bookbinding process selection radio button 1222 is selected (FIG. 12C), the job history information of the cutting/bookbinding process is input to the job history information field 1225.

Since the job history registration screen 1210 is a display screen for inputting the job history of the surface treatment process, the surface treatment process selection radio button 1221 is selected on the job history registration screen 1210. On the other hand, since the job history registration screen 1230 is a display screen for inputting the job history of the cutting/binding process, the cutting/binding process selection radio button 1222 is selected on the job history registration screen 1230. Further, when a cancel button 1223 is pressed, the input contents are discarded and the screen returns to the job history screen 1200. When a registration button 1224 is pressed, the input contents are registered as the job history information.

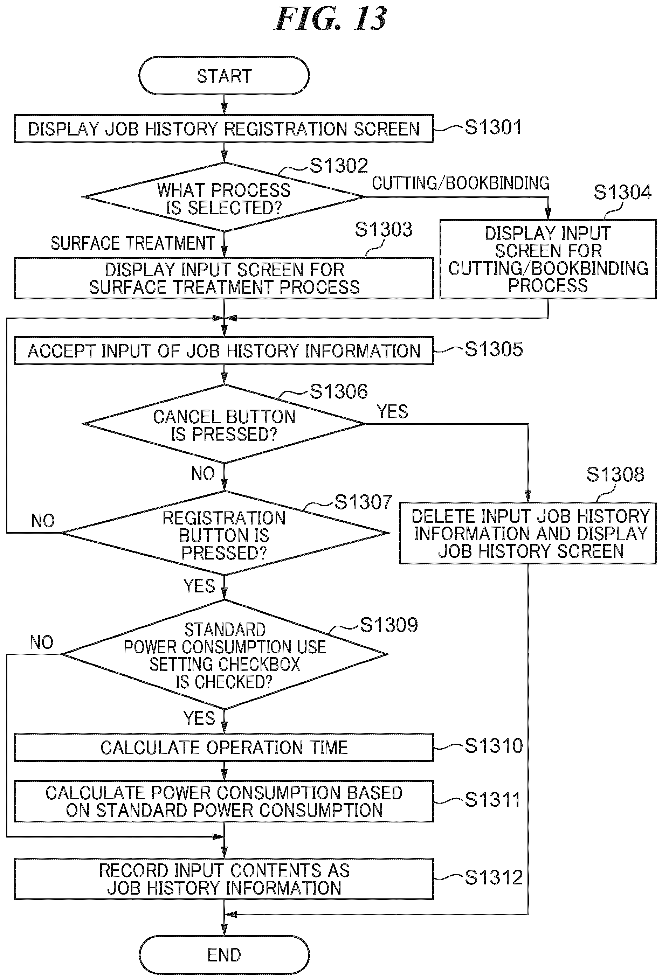

FIG. 13 is a flowchart illustrating a job history registration process executed by the environmental load calculation server. A job history is input in this process. A program based on the flowchart illustrated in FIG. 13 is stored in the HDD 493 of the environmental load calculation server 104. The CPU 491 of the environmental load calculation server 104 loads the program from the HDD 493 onto the memory 492 and executes the program. The program starts when the job history registration button 1201 on the job history screen 1200 is pressed.

As illustrated in FIG. 13, in a step S1301, the CPU 491 generates the job history registration screen 1210 (or the job history registration screen 1230) and displays the generated screen on the display unit 496. After the execution of the step S1301, the process proceeds to a step S1302.

In the step S1302, the CPU 491 determines which of the surface treatment process selection radio button 1221 and the cutting/bookbinding process selection radio button 1222 is selected on the job history registration screen 1210. As a result of the determination in the step S1302, when it is determined that the surface treatment process selection radio button 1221 is selected, the process proceeds to a step S1303. On the other hand, as a result of the determination in the step S1302, it is determined that the cutting/bookbinding process selection radio button 1222 is selected, the process proceeds to a step S1304.

In the step S1303, the CPU 491 generates the job history information field 1225 for entering the job history information of the surface treatment process. After the execution of the step S1303, the process proceeds to a step S1305.

In the step S1304, the CPU 491 generates the job history information field 1225 for inputting the job history information of the cutting/bookbinding process. After the execution of the step S1304, the process proceeds to the step S1305.

In the step S1305, the CPU 491 accepts the job history information input in the job history information field 1225. The CPU 491 stores the job history information in the memory 492. After the execution of the step S1305, the process proceeds to a step S1306.

In the step S1306, the CPU 491 determines whether the cancel button 1223 is pressed. As a result of the determination in the step S1306, when it is determined that the cancel button 1223 is pressed, the process proceeds to a step S1308. On the other hand, as a result of the determination in the step S1306, when it is determined that the cancel button 1223 is not pressed, the process proceeds to a step S1307.

In the step S1307, the CPU 491 determines whether the registration button 1224 is pressed. As a result of the determination in the step S1307, when it is determined that the registration button 1224 is pressed, the process proceeds to a step S1309. On the other hand, as a result of the determination in the step S1307, when it is determined that the registration button 1224 is not pressed, the process returns to the step S1305, and the subsequent steps are executed in order.

If there is a field in which a content of the job history information is not input at the time when it is determined that the registration button 1224 is pressed, the process may return to the step S1305 and the subsequent steps may be executed in order. In addition, when there is a field in which a content of the job history information is not input, a press of the registration button 1224 may be prohibited and a screen indicating the prohibition may be displayed.

In the step S1308, the CPU 491 deletes the job history information stored in the memory 492 in the step S1305 and returns the screen from the job history registration screen 1210 to the job history screen 1200. After the execution of the step S1308, the process ends.

In the step S1309, the CPU 491 determines whether the standard power consumption use setting checkbox 1219 is checked (selected) on the basis of the job history information stored in the memory 492 in the step S1305. As a result of the determination in the step S1309, when it is determined that the standard power consumption use setting checkbox 1219 is checked, the process proceeds to a step S1310. On the other hand, as a result of the determination in the step S1309, when it is determined that the standard power consumption use setting checkbox 1219 is not checked, the process proceeds to a step S1312.

In the step S1310, the CPU 491 calculates the operation time in executing a job. Specifically, the CPU 491 obtains the job start date and time input to the start date field 1213 and the job end date and time input to the end date field 1214, and calculates the elapsed time from the start to the end of the job as the operation time. The operation time is stored in the memory 492. After the execution of the step S1310, the process proceeds to a step S1311.

In the step S1311, the CPU 491 obtains a standard power consumption associated with the machine number input in the machine number field 1211 from an environmental load factor stored in the memory 492 or the **HDD** 493. The CPU 491 calculates the power consumption by multiplying the standard power consumption by the operation time stored in the memory 492 in the step S1310. The power consumption is stored in the memory 492. After the execution of the step S1311, the process proceeds to a step S1312.

In the step S1312, the CPU 491 stores the contents input on the job history registration screen 1210 in the **HDD** 493 as the job history information. After the execution of the step S1312, the process ends.

As described above, in the present embodiment, even if the surface treatment apparatus 102 and the cutting/bookbinding apparatus 103 operate without being connected to the network, the environmental load calculation server 1041 can accept the contents of jobs executed by the apparatuses. This enables to calculate the CO2 emission of the product processed in the job.

Although the contents of the job history are input on the job history registration screen 1210 displayed on the display unit 496 of the environmental load calculation server 104 in the present embodiment, this is not limited. For example, the job history information of the surface treatment apparatus 102 or the cutting/bookbinding apparatus 103 that is not connected to the network may be output in the file format and used. In addition, when the use record of the surface treatment apparatus 102 or the cutting/bookbinding apparatus 103 which is not connected to the network is input as a file, the file may be read and accepted as the input information.

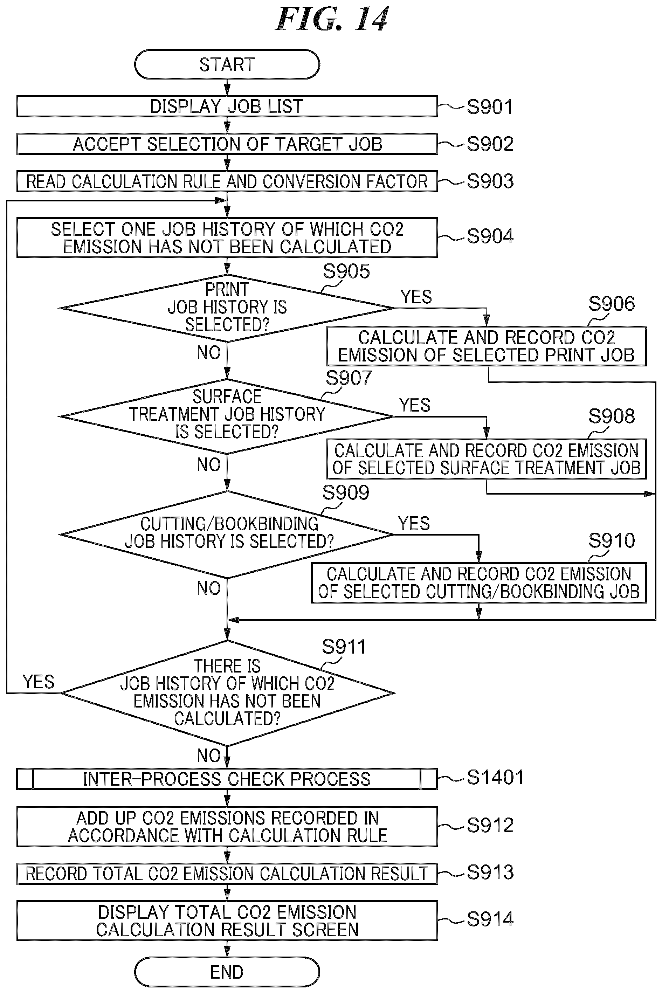

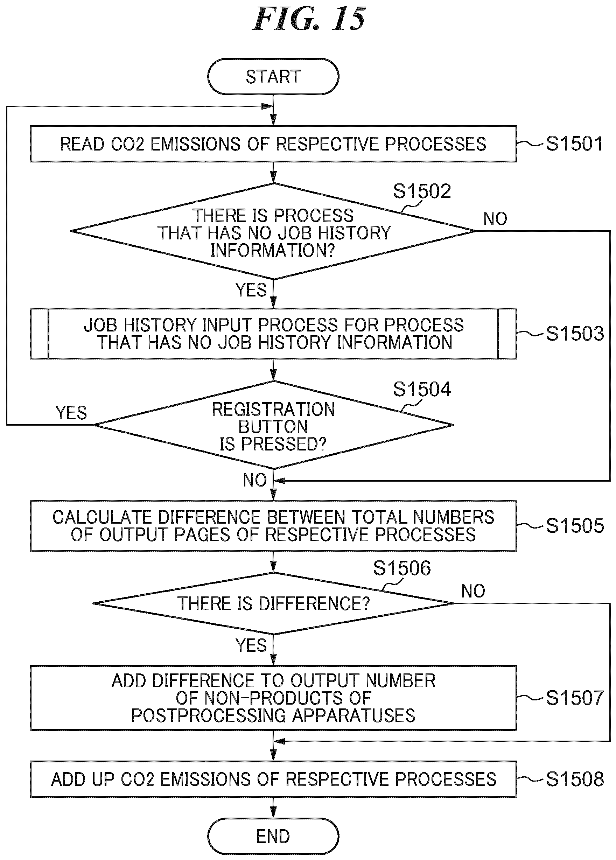

Hereinafter, a third embodiment will be described with reference to FIG. 14 and FIG. 15. Differences from the above-described embodiments will be mainly described, and description of the same matters will be omitted. The image forming apparatus 101 may be operated to produce the product including a spare product in consideration of a case where a problem occurs in the process in the postprocessing apparatus. In this case, when the CO2 emission of the final product is calculated, it is necessary to consider handling of the spare product. In the present embodiment, a configuration in which the environmental load calculation server 104 takes handling of a spare product into consideration will be described.

FIG. 14 is a flowchart illustrating a process executed by the environmental load calculation server 104 related to the third embodiment to calculate a CO2 emission. In the flowchart illustrated in FIG. 14, the steps S901 to S911 are executed in the same manner as in the flowchart illustrated in FIG. 11. Then, as a result of the determination in the step S911, when it is determined that there is no job history of which a CO2 emission has not been calculated, the processing proceeds to a step S1401.

In the step S1401, the CPU 491 reads all the values of the CO2 emissions of the CO2 emission calculation target jobs stored in the HDD 493 and executes an inter-process check process. The inter-process check process will be described later with reference to FIG. 15. After the execution of the step S1401, the steps S912 to S914 similar to the flowchart illustrated in FIG. 11 are executed.

FIG. 15 is a flowchart illustrating the inter-process check process executed in the step 1401 (a subroutine) in the flowchart illustrated in FIG. 14. As shown in FIG. 15, in a step S1501, the CPU 491 reads the CO2 emissions of the respective processes executed. Specifically, the CPU 491 obtains a CO2 emission calculation rule managed by the calculation setting manager 733 and reads all the CO2 emissions to be added up stored in the HDD 493 to the memory 492 in accordance with the CO2 emission calculation rule. The CO2 emission calculation rule is used to determine the CO2 emissions to be added up in calculating the total CO2 emission. When a plurality of jobs are included in one process, the CO2 emissions of the jobs are added up, and the sum is read to the memory 492. After the execution of the step S1501, the process proceeds to a step S1502.

In the step S1502, the CPU 491 determines whether there is a process that has no job history information among the processes of which the CO2 emissions are added up in the step S1501. As a result of the determination in the step S1502, when it is determined that there is a process that has no job history information, the process proceeds to a step S1503. On the other hand, as a result of the determination in the step S1502, when it is determined that there is no process that has no job history information, the process proceeds to a step S1505.

In the step S1503, the CPU 491 executes the job history registration process for the process that is determined to have no job history information in the step S1502. The process in the step S1503 is a subroutine and is performed according to the flowchart illustrated in FIG. 13. That is, the job history registration screen 1210 (FIG. 12B) is displayed on the display unit 496, and when the input to this screen is completed, the process proceeds to a step S1504.

In the step S1504, the CPU 491 determines whether the registration of the job history information is accepted in the step S1503 by determining whether the registration button 1224 on the job history registration screen 1210 to which the input is completed is pressed. As a result of the determination in the step S1504, when it is determined that the registration of the job history information is accepted, the process returns to the step S1501, and the subsequent steps are executed in order. On the other hand, as a result of the determination in the step S1504, when it is determined that the registration of the job history information is not accepted, the process proceeds to the step S1505.

In the step S1505, the CPU 491 reads the total number of output pages and the output number of non-products (wasted spare products) from the total value for each process stored in the memory 492, and calculates difference in the total number of output pages for each process. This calculation is performed on the basis of the total number of output pages of the process in the image forming apparatus 101, the total number of output pages of the process in the postprocessing apparatus, and the output number of non-products.

The comparison between the processes differs depending on the combination of the registered processes. For example, only the print process is executed, the difference is not calculated. When the print process and the surface treatment process are executed or when the print process and the cutting/bookbinding process are executed, a difference between the two processes is calculated. When the print process, the surface treatment process, and the cutting/bookbinding process are executed, a difference between the print process and the surface treatment process and a difference between the surface treatment process and the cutting/bookbinding process are calculated in consideration of a product generation process. The calculated difference is stored in the memory 492. After the execution of the step S1505, the process proceeds to a step S1506.

In the step S1506, the CPU 491 determines whether at least one difference is stored in the memory 492 in the step S1505. As a result of the determination in the step S1506, when it is determined that at least one difference is stored, the process proceeds to a step S1507. On the other hand, as a result of the determination in the step S1506, when it is determined that no difference is stored, the process proceeds to a step S1508.

In the step S1507, the CPU 491 additionally registers the difference between the processes calculated in the step S1505 as the output number of non-products of the process of the postprocessing apparatus in the job history information of the postprocessing apparatus in the HDD 493. After the execution of the step S1507, the process proceeds to the step S1508.

In the step S1508, the CPU 491 reads the calculation target job stored in the HDD 493 at the time of executing the step S1508. Then, the CPU 491 adds up the CO2 emissions of the respective processes of the calculation target job and obtains the total CO2 emission. This total CO2 emission is stored in memory 492. After the execution of the step S1508, the process ends. As described above, in the present embodiment, even when a spare product is generated, the CO2 emission emitted in the manufacturing of the product can be included in the total CO2 emission.

### Other Embodiments

Embodiment(s) of the present disclosure can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)TM), a flash memory device, a memory card, and the like.

While the present disclosure has been described with reference to embodiments, it is to be understood that the present disclosure is not limited to the disclosed embodiments. The scope of the following claims is to be accorded the broadest interpretation so as to encompass all such modifications and equivalent structures and functions.

## Claims

1. An information processing system (1) comprising:
a memory device (492) that stores a set of instructions; and
at least one processor (491) that executes the set of instructions to:
obtain first information (600a to 600f) related to a process until a primary product, which is a printed matter obtained by an image forming apparatus (101) printing on a print medium, is output;
obtain second information (610a to 610e, 620a to 620e) related to a process until a second product, which is obtained by a postprocessing apparatus (102, 103) processing the primary product, is output;
calculate (S906) an emission of greenhouse gas emitted from the image forming apparatus based on the first information;
calculate (S908, S910) an emission of greenhouse gas emitted from the postprocessing apparatus based on the second information; and
calculate (S912) a total emission of the greenhouse gas based on the emission of the greenhouse gas emitted from the image forming apparatus and the emission of the greenhouse gas emitted from the postprocessing apparatus.

2. The information processing system according to claim 1, wherein the at least one processor executes instructions in the memory device to:
obtain at least one first execution history including the first information with respect to the output of the primary product by the image forming apparatus; and
obtain at least one second execution history including the second information with respect to the output of the secondary product by the postprocessing apparatus.

3. The information processing system according to claim 2, wherein the at least one first execution history includes a plurality of first execution histories and at least one second execution history include a plurality of second execution histories, and
wherein the at least one processor executes instructions in the memory device to display a history screen including the plurality of first execution histories and the plurality of second execution histories on a display unit provided in the information processing system.

4. The information processing system according to claim 3, wherein the at least one processor executes instructions in the memory device to accept, prior to a first calculation of calculating an emission of greenhouse gas emitted from the image forming apparatus and the postprocessing apparatus, an operation of selecting a first execution history subjected to the first calculation from among the plurality of first execution histories included in the history screen and an operation of selecting a second execution history subjected to the first calculation from among the plurality of second execution histories included in the history screen.

5. The information processing system according to claim 3, wherein the at least one processor executes instructions in the memory device to:
allow acceptance of an operation of instructing calculation of the emissions of greenhouse gas emitted from the image forming apparatus and the postprocessing apparatus, and acceptance of an operation of instructing calculation of the total emission of greenhouse gas;
display a calculation result of the total emission on the display unit;
accept inputs of the first information and the second information; and
obtain the first information and the second information that are input.

6. The information processing system according to any one of claims 1 to 5, wherein the first information includes at least one of a material consumption and an electric power consumption in a process until the primary product is output by the image forming apparatus,
wherein the second information includes at least one of a material consumption and an electric power consumption in a process until the secondary product is output by the postprocessing apparatus, and
wherein the at least one processor executes instructions in the memory device to:
calculate a first emission of greenhouse gas corresponding to the consumptions included in the first information;
calculate a second emission of greenhouse gas corresponding to the consumptions included in the second information; and
display the first emission calculated and the second emission calculated on a display unit provided in the information processing system.

7. The information processing system according to claim 6, wherein the at least one processor executes instructions in the memory device to:
calculate an emission of greenhouse gas corresponding to the material consumption by multiplying the material consumption by a predetermined coefficient; and
calculate an emission of greenhouse gas corresponding to the electric power consumption by multiplying the electric power consumption by a predetermined coefficient.

8. The information processing system according to any one of claims 1 to 7, wherein the first information includes an amount of waste generated in a process until the primary product is output by the image forming apparatus,
wherein the waste includes a non-product generated in the process until the primary product is output, and
wherein the at least one processor executes instructions in the memory device to:
calculate the emission of greenhouse gas corresponding to the amount of waste; and
display the emission of greenhouse gas calculated corresponding to the amount of waste is displayed on a display unit provided in the information processing system.

9. The information processing system according to any one of claims 1 to 8, wherein the second information includes an amount of waste generated in the process until the secondary product is output by the postprocessing apparatus.

10. The information processing system according to claim 9, wherein the waste includes a surplus part of the secondary product generated by cutting the primary product by the postprocessing apparatus,
wherein the at least one processor executes instructions in the memory device to add a type of the print medium constituting the first product to a calculation condition when an emission of greenhouse gas corresponding to the amount of the surplus part is calculated.

11. The information processing system according to claim 8, wherein the at least one processor executes instructions in the memory device to calculate the emission of greenhouse gas corresponding to the amount of waste by multiplying the amount of waste by a predetermined coefficient.

12. The information processing system according to any one of claims 1 to 11, wherein the at least one processor executes instructions in the memory device to:
calculate a CO2 emission emitted from the image forming apparatus as the emission of greenhouse gas emitted from the image forming apparatus; and
calculate a CO2 emission emitted from the postprocessing apparatus as the emission of greenhouse gas emitted from the processing apparatus.

13. The information processing system according to any one of claims 1 to 12, wherein at least one of an apparatus that performs treatment on a surface of the primary product and an apparatus that performs cutting of the primary product.

14. A control method for an information processing system, the control method comprising:
obtaining first information related to a process until a primary product, which is a printed matter obtained by an image forming apparatus printing on a print medium, is output;
obtaining second information related to a process until a second product, which is obtained by a postprocessing apparatus processing the primary product, is output;
calculating an emission of greenhouse gas emitted from the image forming apparatus based on the first information;
calculating an emission of greenhouse gas emitted from the postprocessing apparatus based on the second information; and
calculating a total emission of the greenhouse gas based on the emission of the greenhouse gas emitted from the image forming apparatus and the emission of the greenhouse gas emitted from the postprocessing apparatus.

15. A computer program comprising instructions which, when the program is executed by a computer, cause the computer to carry out the method of claim 14.
